(19) Europäisches Patentamt
European Patent Office
Office européen des brevets

(11) Publication number: **0 298 135 A1**

(12) # EUROPEAN PATENT APPLICATION

(21) Application number: 87104691.8

(22) Date of filing: 30.03.87

(51) Int. Cl.4: C07C 129/12, C07C 103/80, C07C 103/84, C07C 123/00, C07D 213/32, C07D 213/40, C07D 213/75, C07D 295/18

(43) Date of publication of application: 11.01.89 Bulletin 89/02

(84) Designated Contracting States: BE CH DE FR GB IT LI NL SE

(71) Applicant: Okamoto, Shosuke
15-18, Asahigaoka 3-chome Tarumi-ku
Kobe-shi Hyogo(JP)

Applicant: Showa Denko Kabushiki Kaisha
10-12, Shiba Daimon 2-chome Minato-ku
Tokyo 105(JP)

(72) Inventor: Okamoto, Shosuke
15-18, Asahigaoka 3-chome Tarumi-ku
Kobe-shi Hyogo(JP)
Inventor: Okada, Yoshio
542-1, Aza Shimizu Okuradani
Akashi-shi Hyogo(JP)
Inventor: Okunomiya, Akiko
13-2, Nakayamatedori 7-chome Chuo-ku
Kobe-shi Hyogo(JP)
Inventor: Naito, Taketoshi
Showa Denko K.K. Seikagakukenkyusho, 24-25
Tamagawa 2-chome Ohta-ku Tokyo(JP)
Inventor: Kimura, Yoshio
Showa Denko K.K. Seikagakukenkyusho, 24-25
Tamagawa 2-chome Ohta-ku Tokyo(JP)
Inventor: Yamada, Morihiko
Showa Denko K.K. Seikagakukenkyusho, 24-25
Tamagawa 2-chome Ohta-ku Tokyo(JP)
Inventor: Ohno, Norio
Showa Denko K.K. Seikagakukenkyusho, 24-25
Tamagawa 2-chome Ohta-ku Tokyo(JP)
Inventor: Katsuura, Yasuhiro
Showa Denko K.K. Seikagakukenkyusho, 24-25
Tamagawa 2-chome Ohta-ku Tokyo(JP)

EP 0 298 135 A1

Xerox Copy Centre

Inventor: **Nojima, Hiroshi**
**Showa Denko K.K. Seikagakukenkyusho, 24-25**
**Tamagawa 2-chome Ohta-ku Tokyo(JP)**
Inventor: **Shishikura, Takashi**
**Showa Denko K.K. Seikagakukenkyusho, 24-25**
**Tamagawa 2-chome Ohta-ku Tokyo(JP)**

(74) Representative: **Strehl, Schübel-Hopf, Groening, Schulz**
**Widenmayerstrasse 17 Postfach 22 03 45**
**D-8000 München 22(DE)**

(54) **Phenylalanine derivative and proteinase inhibitor.**

(57) A phenylalanine derivative having the formula (I):

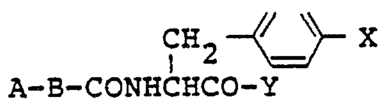

(I)

wherein A represents

(a) $H_2N-$, (b) 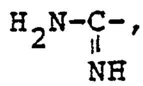, or (c) 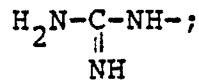;

B represents (a) 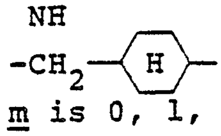, (b) 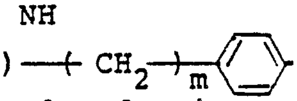

or (c) $-(CH_2)_n-$, wherein m is 0, 1, 2, or 3 and n is 3, 4, or 5, provided that A-B is not 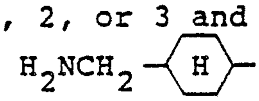, 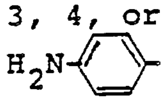, and 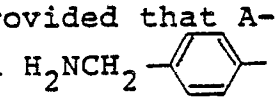;

X represents hydrogen, (b) hydroxy, (c) nitro, or (d) $C_1$-$C_4$ alkyloxy which may be substituted with phenyl;

Y represents

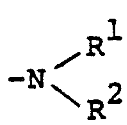

wherein $R^1$ and $R^2$ are, independently, (a) hydrogen, provided that both $R^1$ and $R^2$ are not hydrogen at the same time; (b) phenyl, which may be substituted with (i) phenylcarbonyl, (ii) $C_1$-$C_4$ alkylcarbonyl, (iii) $C_1$-$C_4$ alkyl, which may be further substituted with $C_1$-$C_4$ alkoxycarbonyl or hydroxycarbonyl, (iv) $C_2$-$C_5$ alkenyl, which may further be substituted with $C_1$-$C_4$ alkoxycarbonyl, (v) trifluoromethyl; (c) pyridyl, which may be substituted with $C_1$-$C_4$ alkoxy; (d) $C_1$-$C_4$ alkyl, which may be substituted with (i) hydroxy, (ii) $C_1$-$C_4$ alkoxycarbonyl, (iii) pyridyl, or (iv) phenyl, which may be further substituted with $C_1$-$C_4$ alkylaminocarbonyl; (e) $C_5$-$C_7$ cycloalkyl, which may be substituted with $C_1$-$C_4$ alkyl; or (f) $R^1$ and $R^2$ may form, with the nitrogen atom attached thereto, (i) piperidino, which may be substituted with phenylcarbonyl or (ii) pyrrolidyl, which may be substituted with $C_1$-$C_4$ alkoxycarbonyl; or a pharmaceutically acceptable salt thereof.

This phenylalanine derivative is effective as a proteinase inhibitor.

# PHENYLALANINE DERIVATIVE AND PROTEINASE INHIBITOR

## BACKGROUND OF THE INVENTION

### I. Field of the Invention

The present invention relates to a novel phenylalanine derivative, more particularly to a phenylalanine derivative having a proteinase inhibition activity or a pharmaceutically acceptable salt thereof. The present invention also relates to a proteinase inhibitor containing the same as the effective ingredient.

### 2. Description of the Related Art

It is well known in the art that various proteinases are present in human organisms, for example, plasmin, trypsin, kallikrein, urokinase, and the like. As is also known, when these proteinases are abnormally activated for some reason, various diseases are caused. For example, hemorrhagic diseases are caused when a relatively large amount of abnormally activated plasmin is present in the blood. Also, plasmin is an inflammatory agent and is considered to cause inflammatory diseases. Therefore, a substance capable of exhibiting a proteinase inhibition activity is useful as a clinical remedy or medicine, and various investigations have been made in the prior art into the development of such substances. For example, antiplasmins are useful as hematostatic agents, anti-inflammatory agents or anti-allergic agents, antitrypsins are useful for the therapy of pancreatitis, antikallikreins are useful as therapeutical agents for inflammation, and anti-urokinases are useful for the inhibition of hemorrhagic symptoms in the thrombolytic therapeutical method with urokinase. Accordingly, developments have been made in the prior art of proteinase inhibitors having such activities, but the proteinase inhibition activities are low and are not satisfactory for practical application as medicines. Namely, compounds having satisfactory inhibition activities against various proteinases have not been developed.

## SUMMARY OF THE INVENTION

Accordingly, the objects of the present invention are to eliminate the above-mentioned disadvantages of the prior art and to provide a compound having a satisfactory inhibition activity in practical application, and in addition, still having satisfactory inhibition activities against various proteinases, and a proteinase inhibitor containing the compound as the effective ingredient.

Other objects are advantages of the present invention will be apparent from the following description.

In accordance with the present invention, there is provided a phenylalanine derivative having the formula (I):

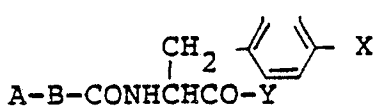

(I)

wherein A represents

(a) $H_2N-$, (b) 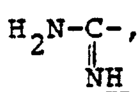, or (c) 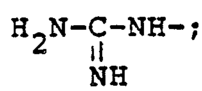;

B represents (a) 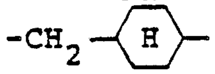, (b) 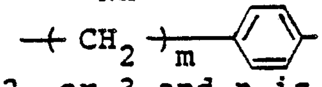 or (c) $-(CH_2)_n-$, wherein m is 0, 1, 2, or 3 and n is 3, 4, or 5, provided that A-B is not 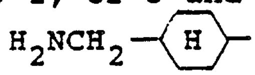, 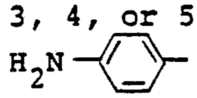, and 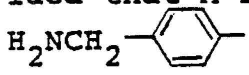;

X represents (a) hydrogen, (b) hydroxy, (c) nitro, or (d) $C_1$-$C_4$ alkyloxy which may be substituted with phenyl;

Y represents

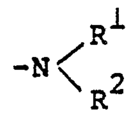

wherein $R^1$ and $R^2$ are, independently, (a) hydrogen provided that both $R^1$ and $R^2$ are not hydrogen at the same time; (b) phenyl which may be substituted with (i) phenylcarbonyl, (ii) $C_1$-$C_4$ alkylcarboxyl, (iii) $C_1$-$C_4$ alkyl which may be further substituted with $C_1$-$C_4$ alkoxycarbonyl or hydroxycarbonyl, (iv) $C_2$-$C_5$ alkenyl which may further be substituted with $C_1$-$C_4$ alkoxycarbonyl, (v) trifluoromethyl; (c) pyridyl, which may be substituted with $C_1$-$C_4$ alkoxy; (d) $C_1$-$C_4$ alkyl which may be substituted with (i) hydroxy, (ii) $C_1$-$C_4$ alkoxycarbonyl, (iii) pyridyl, or (iv) phenyl which may be further substituted with $C_1$-$C_4$ alkylaminocarbonyl; (e) $C_5$-$C_7$ cycloalkyl, which may be substituted with $C_1$-$C_4$ alkyl; or (f) $R^1$ and $R^2$ may form, with the nitrogen atom attached thereto, (i) piperidino which may be substituted with phenylcarbonyl or (ii) pyrrolidyl, which may be substituted with $C_1$-$C_4$ alkoxycarbonyl; or a pharmaceutically acceptable salt thereof.

Examples of such a salt may include inorganic acid salts such as hydrochloride, hydrobromide, hydroiodide, sulfate, nitrate, phosphate, etc.; organic acid salts such as oxalate, succinate, glycolate, malate, citrate, maleate, lactate, benzenesulfonate, toluenesulfonate, methanesulfonate, etc.

In accordance with the present invention, there is also provided a proteinase inhibitor comprising the phenylalanine derivative of the above formula (I) or a pharmaceutically acceptable salt thereof as the active ingredient.

## DESCRIPTION OF THE PREFERRED EMBODIMENTS

The compounds according to the present invention have phenylalanine as a basic skeleton as shown in the general formula (I) and have characteristic structure in which amino, amidino, or guanidino group (i.e., group A) is attached to the N-terminal of the amino group of the phenylalanine by a peptide linkage via the hydrocarbon group (i.e., group B) having certain size. The preferable combination of the groups A and B (i.e., A-B) are as follows.

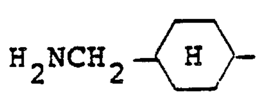 , 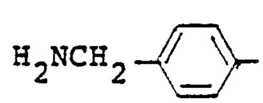 , $H_2N(CH_2)_{3-5}-$ ,

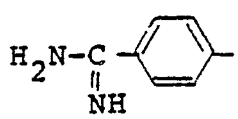 , 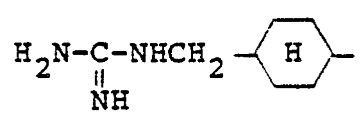 ,

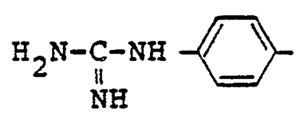 , 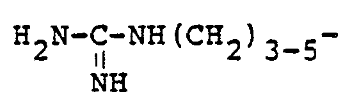

of these combinations, the following is especially preferable.

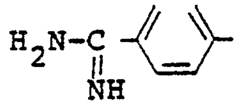 or 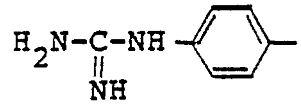

On the other hand, to the terminal carbon of the carboxyl group of phenylalanine, as the Y group, various substituents are bonded via the peptide linkage. Furthermore, specified substituents (e.g., group X) are introduced at 4-position (i.e., para position) for the benzene ring of the phenylalanine. Among various substituents in the X and Y groups, the following is especially preferable in the combination with the above mentioned A-B group.

X: hydrogen, nitro or benzyloxy and

Y: -NHR wherein R is (a) phenyl which may be substituted with (i) phenylcarbonyl, (ii) $C_1$-$C_4$ alkylcarboxyl, (iii) $C_1$-$C_3$ alkyl which may furhter be substituted with $C_1$-$C_2$ alkoxycarbonyl or hydroxycarbonyl, (iv) $C_2$-$C_5$ alkenyl which may further be substituted with $C_1$-$C_2$ alkoxycarbonyl; (b) pyridyl which may be substituted with $C_1$ alkoxy; or (c) $C_1$-$C_4$ alkyl which may be substituted with pyridyl, or phenyl which may further be substituted with di $C_1$ alkylaminocarbonyl; or piperidino which may be substituted with phenylcarbonyl.

Typical examples of the compound represented by the above formula are listed in Table I.

The compounds listed in the Table are numbered, respectively, and in the following description, the individual compounds are designated in terms of said compound Nos. for convenience. For the compounds medicated as L in the chemical structure, this means that their asymetric carbons are L forms. All of asymetric carbons in phenylalanine skelton in the compounds listed in table I are L form.

In the physical properties shown in Table I, NMR represents a nuclear magnetic resonance spectrum indicated by $\delta$ (i.e., delta) (ppm) representing the chemical shifts. The determination was carried out by using as a solvent $CDCl_3$ (i.e., heavy chloroform), $(CD_3)_2SO$ (i.e., $d_6$-dimethylsulfoxide), $D_2O$ (i.e., heavy water), or $CD_3OD$ (i.e., heavy methanol) alone or in any mixture thereof, and by using as an internal standard TMS (i.e., tetramethylsilane). In the parenthesis after the $\delta$ number, the number of the hydrogen atom and the symbols s, d, t, q, m, and broad, hereinafter, denote singlet, doublet, triplet, quartet, multiplet, and broad absorbance, respectively. The absorbance based on the solvent is not given in the Table.

IR represents an infrared absorption spectrum in which a potassium bromide tablet is used in the determination unless otherwise noted. When a solution is used in the determination, the kind of solvent is listed in parenthesis. The number listed in the Table represents a wave number in units of $cm^{-1}$, and only the main absorption peaks are listed in the Table.

MS represents a mass spectrum, and the results are shown as M/e (i.e., the mass of the cation fragment divided by the charge) of the main peaks.

Table 1

<table>
<tr><th>No.</th><th>Compound</th><th colspan="2">Physical Properties</th></tr>
<tr><td>1</td><td>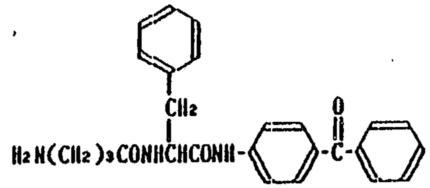</td><td>NMR:<br>CDCl$_3$,TMS<br>δ 1.50--1.80(2H,m)<br>2.10--2.30(2H,m)<br>2.40--2.80(4H,m)<br>2.80--3.30(2H,m)<br>4.80--5.00(1H,m)<br>7.00--7.60(14H,m)</td><td></td></tr>
<tr><td>2</td><td>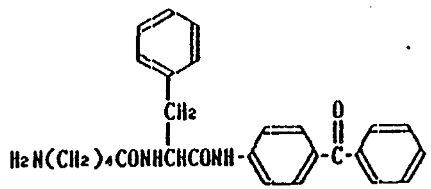</td><td>MS:<br>M/e 443,344,327,<br>253,236,197</td><td>NMR:<br>CDCl$_3$,TMS<br>δ 1.20--1.70(4H,m)<br>1.90--2.30(4H,m)<br>2.50--2.70(2H,m)<br>2.90--3.30(2H,m)<br>4.80--5.00(1H,m)<br>7.00--7.80(14H,m)</td></tr>
<tr><td>3</td><td>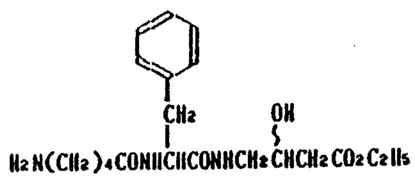</td><td>NMR:<br>CDCl$_3$,TMS<br>δ 1.10--1.70(7H,m)<br>2.10--2.30(4H,m)<br>2.50--2.70(2H,m)<br>2.80--3.40(6H,m)<br>4.00--4.20(2H,q)<br>4.60--4.90(1H,broad)<br>5.10--5.30(1H,broad)<br>7.10--7.30(5H,m)</td><td></td></tr>
<tr><td>4</td><td>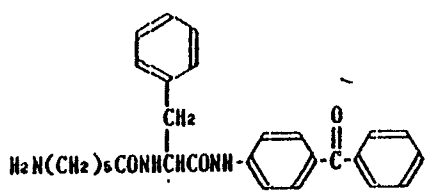</td><td>MS:<br>M/e 457,328,253,197</td><td>NMR:<br>CDCl$_3$, TMS<br>δ 1.00--1.70(6H,m)<br>1.90--2.30(2H,broad)<br>2.50--2.70(2H,broad)<br>2.90--3.40(2H,broad)<br>4.90--5.20(1H,m)<br>7.10--7.80(14H,m)</td></tr>
</table>

**Table 1 (Continued)**

| | | | |
|---|---|---|---|
| 5 | 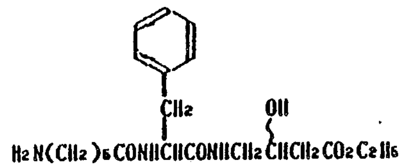 | NMR:<br>CDCl₃, TMS<br>δ 1.10--1.70(9H,m)<br>2.00--2.20(4H,m)<br>2.60--2.80(2H,broad)<br>2.80--3.60(6H,m)<br>4.00--4.20(2H,q)<br>4.60--4.70(1H,m)<br>5.00--5.20(1H,m)<br>6.90--7.40(5H,m) | |
| 6 | 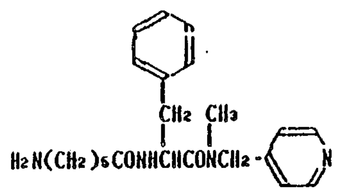 • 2HCl | IR:<br>3400,2930,1640,1510,<br>1500,1455,1365,1250,<br>1200,1120,1025, 790,<br>750, 700 | |
| 7 | 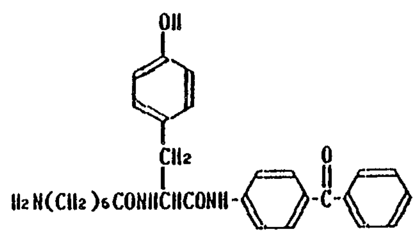 | MS:<br>M/e 473,360,344,276,<br>197 | NMR:<br>$CD_3OD-CDCl_3$, TMS<br>δ 1.10--1.70(6H,m)<br>2.10--2.30(2H,m)<br>2.60--2.70(2H,m)<br>3.00--3.20(1H,m)<br>4.60--4.80(1H,m)<br>6.74--7.06(4H,q)<br>7.50--7.80(9H,m) |

Table 1 (Continued)

| | | | |
|---|---|---|---|
| 8 | 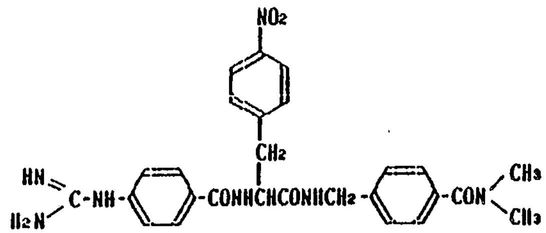 • $CH_3SO_3H$ | IR: 3400,2930,1640,1605, 1560,1515,1345,1205, 1190,1055,1045, 860, 780 | |
| 9 | 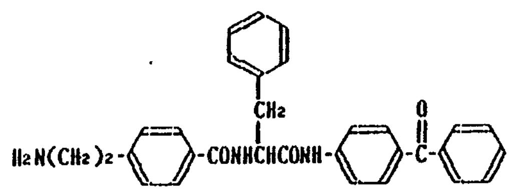 | NMR: $CDCl_3$,TMS δ 2.80--3.40(6H,m) 5.00--5.10(1H,m) 7.00--7.90(18H,m) | |
| 10 | 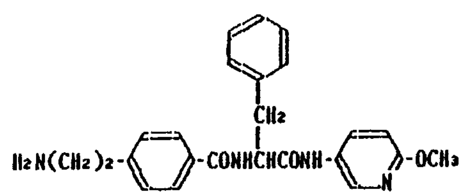 • 2HCl | IR: 3430,3030,1645,1615, 1550,1500,1440,1335, 1300,1180,1150,1015, 850, 745, 700 | |

Table 1 (Continued)

| | | | |
|---|---|---|---|
| 11 | 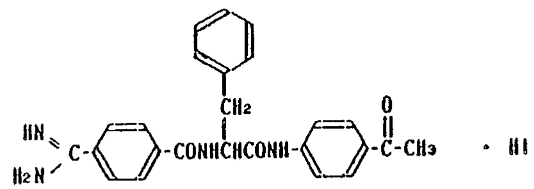 | IR:<br>3300,3210,2960,2930,<br>2870,1648,1638,1535,<br>1480,1415,1245,1015,<br>885, 860 | |
| 12 | 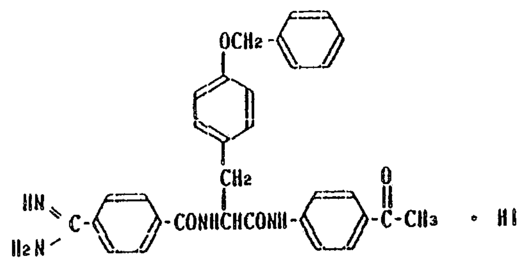 | IR:<br>3700-2000,1660,1590,<br>1530,1510,1480,1400,<br>1300,1270,1240,1175,<br>1010, 835, 730, 695 | NMR:<br>$CD_3OD$,TMS<br>δ 2.52 (3H,s)<br>3.20 (2H,m)<br>4.96 (2H,s)<br>6.84--8.00(17H,m) |
| 13 | 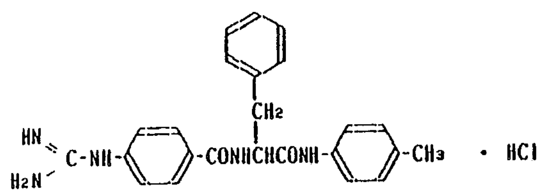 | MS:<br>M/e 373,353,267,239,<br>107 | NMR:<br>$CD_3OD$,TMS<br>δ 1.00--4.00(4H,broad)<br>1.12--1.24(2H,m)<br>2.30 (3H,s)<br>3.04--3.28(2H,m)<br>4.08--4.40(1H,broad)<br>7.04--7.92(13H,m) |

Table 1 (Continued)

| | | | |
|---|---|---|---|
| 14 | 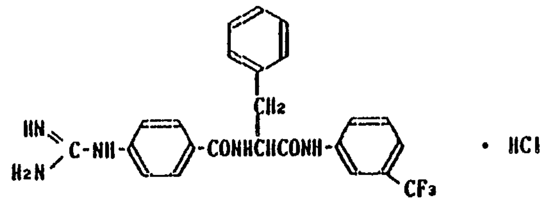 | IR: 3400, 2960, 1675, 1645, 1605, 1565, 1500, 1450, 1330, 1165, 1125, 1070, 800, 700 | |
| 15 | 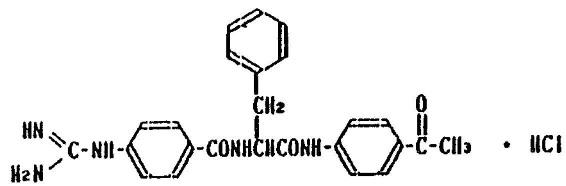 | IR: 3420, 3190, 1675, 1600, 1575, 1535, 1505, 1410, 1360, 1310, 1270, 1255, 1180, 1020, 960, 840, 740, 700 | |
| 16 | 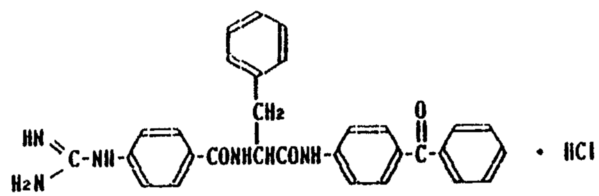 | IR: 3400, 3180, 1645, 1600, 1530, 1500, 1410, 1310, 1280, 1250, 1175, 740, 700 | |

Table 1 (Continued)

| | | | |
|---|---|---|---|
| 17 | 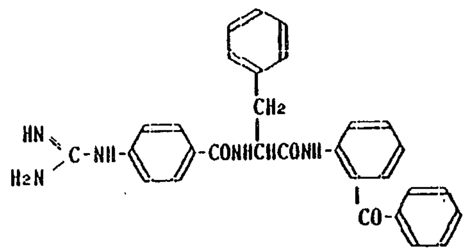 • HCl | IR: 3420,2970,1645,1605, 1580,1535,1505,1450, 1320;1295,1260,1160, 940, 750, 700 | |
| 18 | 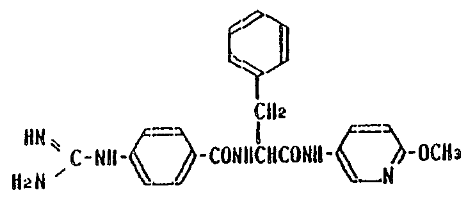 • 2HCl | IR: 3400,3050,1680,1645, 1605,1560,1505,1440, 1300,1115, 840, 740, 700 | |
| 19 | 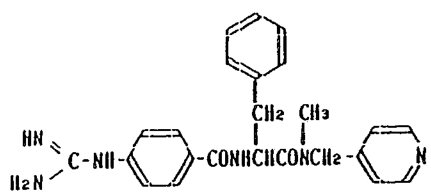 • $CH_3SO_3H$ | MS: M/e 431,341,309,281, 152 | IR: 3400,2930,1640,1605, 1565,1500,1420,1310, 1200,1040, 780, 700 |

**Table 1 (Continued)**

| | | | |
|---|---|---|---|
| 20 | 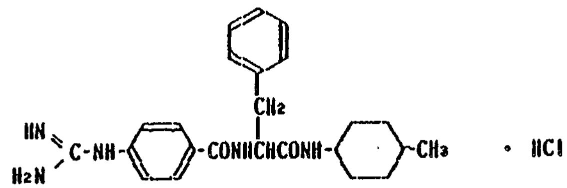 | MS:<br>M/e 379,288,266,244,<br>240,224,136,120,<br>99 | NMR:<br>$CD_3OD$,TMS<br>$\delta$ 0.52--1.90(11H,m)<br>2.92--3.88(6H,m)<br>4.24--4.76(1H,broad)<br>6.96--7.60(4H,m)<br>8.08--8.80(2H,broad) |
| 21 | 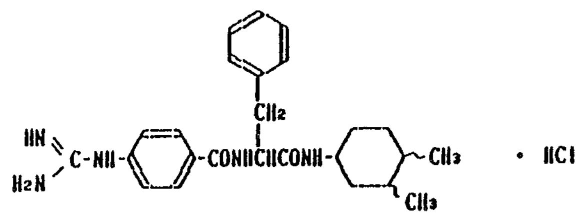 | IR:<br>3420,2960,2940,1645,<br>1605,1570,1505,1455,<br>1255,1115, 700 | |
| 22 | 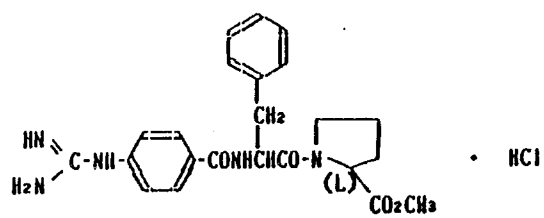 | IR:<br>3370,3170,2980,1740,<br>1680,1630,1600,1565,<br>1500,1450,1300,1280,<br>1275, 760, 700 | |

Table I (Continued)

| | | | |
|---|---|---|---|
| 23 | 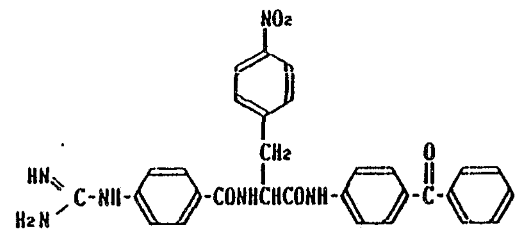 • $CH_3SO_3H$ | IR: 3200,1635,1600,1520, 1340,1200,1170,1040 | |
| 24 | 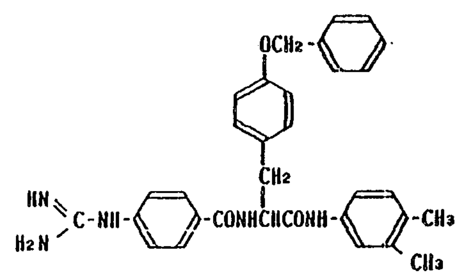 • HCl | IR: 3420,2925,1675,1645, 1605,1540,1510,1455, 1300,1240,1180,1020, 820, 740, 695 | |
| 25 | 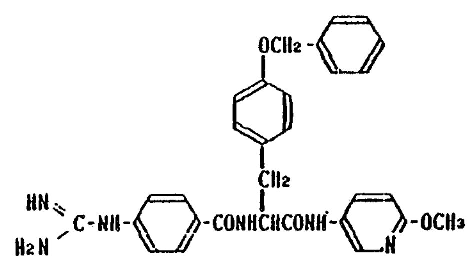 • 2HCl | IR: 3400,3030,1675,1640, 1555,1510,1440,1300, 1240,1180,1010, 830, 700 | |

**Table I (Continued)**

| | | | |
|---|---|---|---|
| 26 | 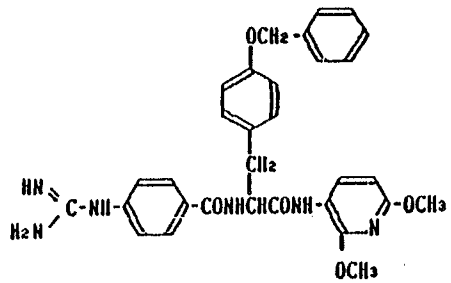 • 2HCl | MS:<br>M/e 526,390,282,197,154 | IR:<br>3320,2980,1640,1605,1570,1510,1480,1420,1390,1300,1245,1180,1100,1015, 810, 735, 700 |
| 27 | 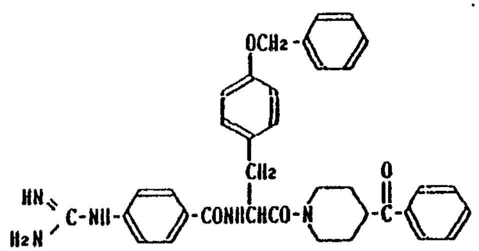 • HCl | MS:<br>M/e 425,408,350,238,211,197,189,134 | IR:<br>3320,2980,1640,1605,1570,1510,1480,1420,1390,1300,1245,1180,1100,1015, 810, 735, 700 |
| 28 | 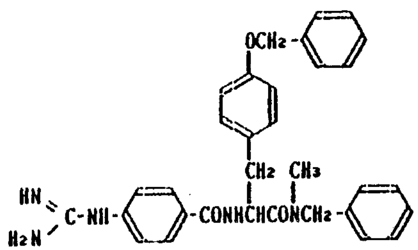 • $CH_3SO_3H$ | MS:<br>M/e 536,387,226,162 | IR:<br>3400,3060,3040,2930,1630,1570,1510,1455,1300,1235,1200,1040, 840, 780, 730, 700 |

Table 1 (Continued)

| | | | |
|---|---|---|---|
| 29 | 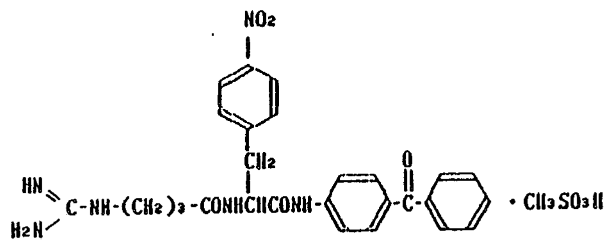 | IR: 3200,1650,1600,1520, 1340,1170,1040, 850, 780 | |
| 30 | 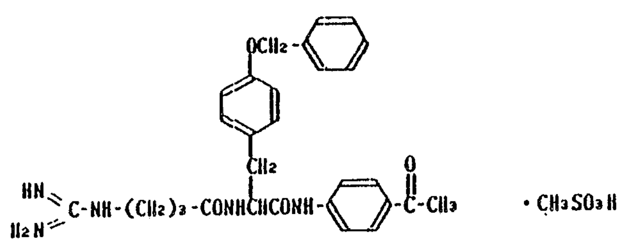 | MS: M/e 516,226,162,128 | IR: 3420,2930,1660,1600 1510,1205,1195,1045 790, 695 |
| 31 | 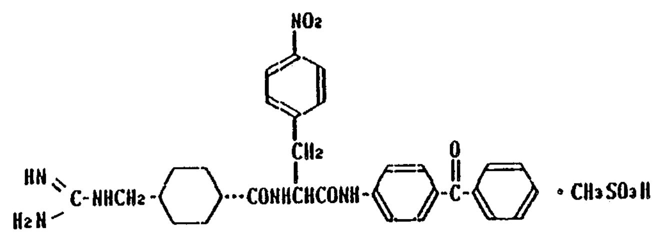 | IR: 3350,1650,1595,1520, 1350,1170,1040 | |

**Table 1 (Continued)**

| | | | |
|---|---|---|---|
| 32 | 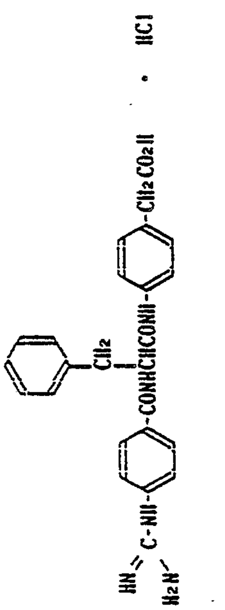 | NMR: $CD_3OD$,TMS δ 1.28 (1H,s) 2.02--3.92(6H,m) 4.24--4.76(1H,broad) 7.04--8.80(13H,m) | |
| 33 | 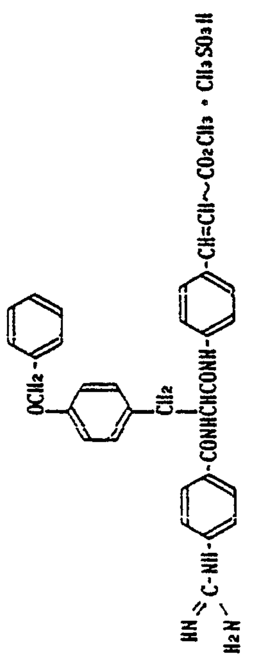 | IR: 3420,3180,2950,1680, 1640,1600,1510,1310 1205,1195,1045, 835, 780, 695 | |
| 34 | 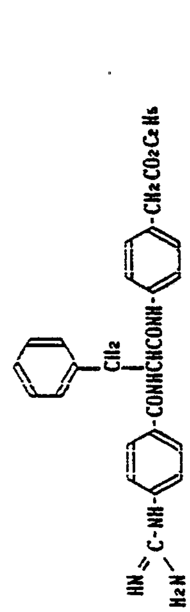 | NMR $CD_3OD$,TMS δ 1.00--1.24(4H,m) 3.28--3.48(2H,m) 4.78--4.96(1H,broad) 7.08--7.88(13H,m) | |

The compounds of the present invention can be synthesized by various combinations of the so-called peptide synthesis methods.

1) Mixed acid anhydride method [Ann, Chem., 572,] 190 (1951)

2) Acid chloride method [Biochemistry, 4, 2219 (1965)]

3) Phosphazo method [Chem. Ber., 93, 2387 (1960)]

4) Carbodiimide method [J. Am. Chem. Soc., 77, 1067 (1955), J. Am. Chem. Soc., 84 4457 (1962), Biochem. Biophy. Res. Comm., 52 No3 (1973), Chem. Ber., 103 2034 (1970), Tet. Lett., 46 4475 (1978).]

5) Active ester method using, for example, N-hydroxysuccinimide [J. Am. Chem. Soc., 85, 3039 (1963)].

It should be noted that not all of the compounds can be synthesized according to the methods mentioned herein, in that it is necessary to combine the above-mentioned methods appropriately for the respective compounds. Among these methods, typical examples of the reaction routes are shown below.

Route A

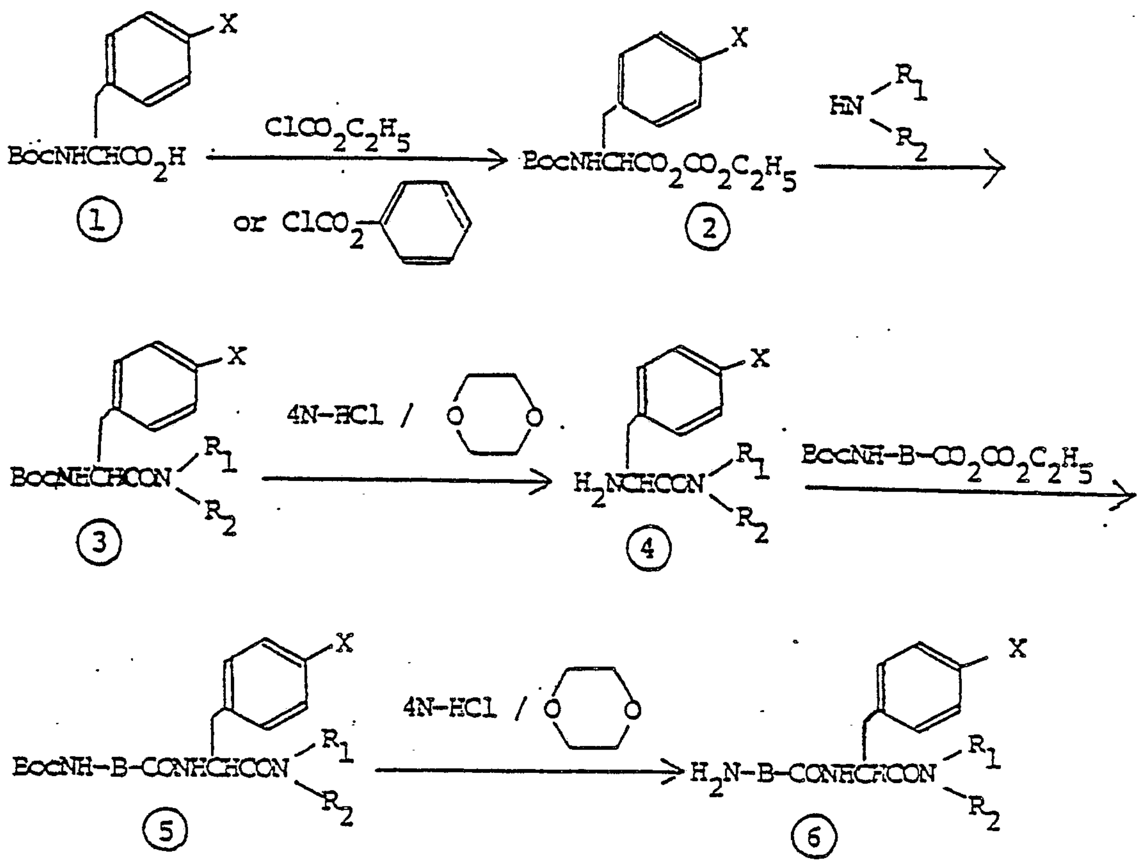


Boc: t-butyloxycarbonyl

For carrying out synthesis from ① to ③, ① is dissolved in an appropriate solvent such as THF, dimethylsulfoxide diethyl ether, dioxane, and the like, and an appropriate base such as triethylamine, pyridine, and the like, is added in an amount of l equivalent to 5 equivalents, preferably 2 to 3 equivalents, relative to ①. To this reaction mixture is added ethyl chlorocarbonate as such, or as a solution dissolved in the solvent used as the reaction solvent, at one time or in several divided portions. The temperature of the reaction mixture is maintained at -l0 °C to 30 °C, preferably 5 to l0 °C. The reaction time is from l hour to 50 hours, preferably from 5 to 20 hours. After a conventional post-treatment, 0.5 to 2 equivalents of

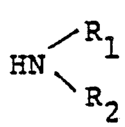

are added and the reaction is carried out at -l0 °C to 30 °C, preferably 5 to 20 °C, for l to 50 hours, preferably 5 to 20 hours. Then, after a conventional post-treatment, ③ is obtained.

The reaction from ③ to ④ may be carried out by allowing ③ to react with 1 to 10 equivalents, preferably 3 to 7 equivalents relative to ③, of 4N-HCl dioxane solution at room temperature. Then, after a conventional post-treatment, ④ is obtained. The reactions from ④ to ⑥ can be carried out in the same way as from ① to ④, whereby ⑥ can be obtained.

Route B

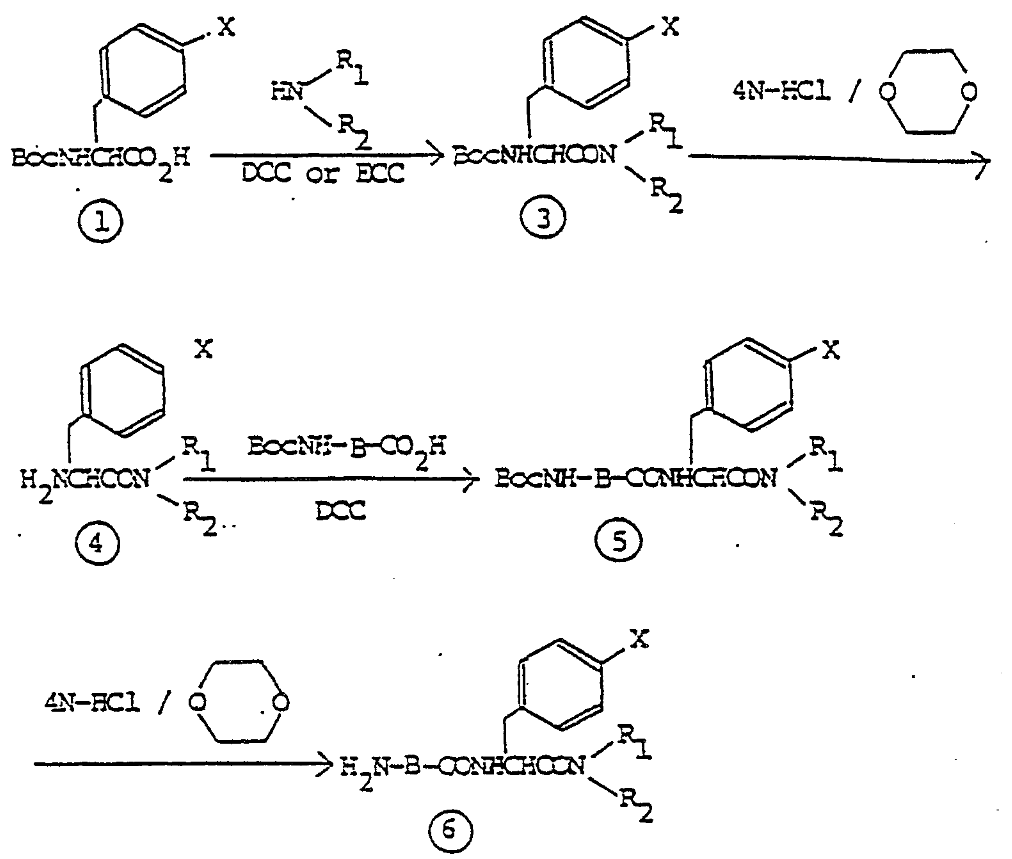


DCC: N,N'-dicyclohexylcarbodiimide

ECC: 1-Ethyl-3-(3-dimethylammopropyl)carbodiimide hydrochloride

Route C

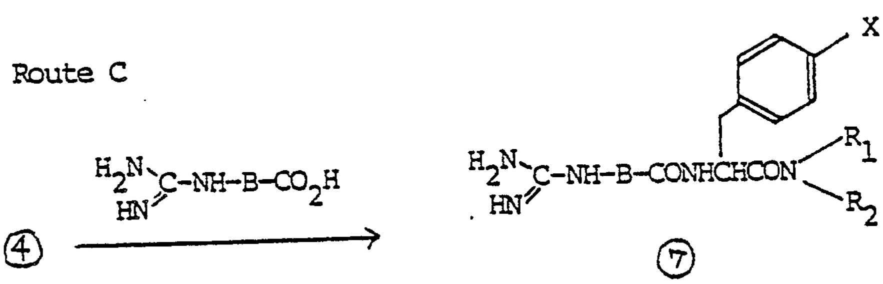


For syntheses from ① to ③, from ④ to ⑤ and from ④ to ⑦, there may be employed, for example, the methods described in J. Am. Chem. Soc., 77 1067 (1955.). For the reactions from ③ to ④ and from ⑤ to ⑥, the methods described in route A may be used.

## EXAMPLES

The present invention will now be further illustrated by, but is by no means limited to, the following Examples. In the following, the preparation of typical compounds is described by referring to specific examples.

### Example I

#### Synthesis of N-(5-aminovaleryl)-L-phenylalanine 4-benzoylanilide (Compound No. 2)

5-Aminovaleric acid hydrochloride (3 g) and sodium bicarbonate (8.5 g) were dissolved in water (30 ml) and a toluene solution of benzyl chlorocarbonate (I3.3 g) dissolved in diethyl ether (I0 ml) was dropwise added to the solution under ice-cooling over I5 minutes, followed by stirring at room temperature for I2 hours. The resultant reaction mixture was washed several times with diethyl ether and was neutralized with I0% hydrochloric acid solution, followed by extracting with ethyl acetate. After the extract was dried over sodium sulfate, the solvent was distilled off. The residue was recrystallized from methylene chloride to obtain 5-benzyloxycarbonylaminovaleric acid (4.I g) in the form of a white solid.

N-(t-butyloxycarbonyl)-L-phenylalanine (I g) was dissolved in dry tetrahydrofuran (30 ml) and triethylamine (2 ml) and ethyl chlorocarbonate (0.45 g) were added thereto under ice-cooling, followed by stirring for 30 minutes. To this solution, 4-benzoylanilide (0.68 g) was added and the mixture was stirred at room temperature for I0 hours. After distilling off the solvent, the resultant reaction mixture was extracted with ethyl acetate and was washed with water, followed by drying over sodium sulfate. The solvent was distilled off and the residue was recrystallized from chloroform. Thus, N-(t-butyloxycarbonyl)-L-phenylalanine-4-benzoylanilide (I) (I.2 g) was obtained in the form of a white solid, which was confirmed by NMR and IR analyses.

To the compound (I) (0.8 g) obtained above, 4N-hydrogen chloride/dioxane solution (6 ml) was added under ice-cooling, followed by stirring at room temperature for 30 minutes. Hexane (20 ml) was added thereto and the precipitated solid was washed several times with diethyl ether to obtain L-phenylalanine 4-benzoylanilide hydrochloride (II) (0.6 g) in the form of a white solid, which was confirmed by an NMR analysis.

The compound (II) (0.5 g) and 5-benzyloxycarbonylaminovaleric acid (0.34 g) were dissolved in dry pyridine and the condensation agent, I-ethyl-3-(3-dimethylaminopropyl)carbodiimide hydrochloride (0.3 g) was added under ice-cooling, followed by stirring at room temperature for I2 hours. After removing the solvent, the residue was extracted with methylene chloride, followed by washing with water. After drying over sodium sulfate, the solvent was distilled off and the residue was recrystallized from methylene chloride to obtain N-(5-benzyloxycarbonylaminovaleryl)-L-phenylalanine 4-benzoylanilide (III) (0.6 g) in the form of a pale yellow solid, which was confirmed by NMR and IR analyses.

The compound (III) (0.6 g) obtained above was added to 30% hydrobromic acid/acetic acid solution (5 ml) under ice-cooling, followed by stirring for 20 minutes. Then, diethyl ether (30 ml) was added and the precipitated solid was washed several times with diethyl ether. A saturated sodium bicarbonate solution (I0 ml) was then added under ice-cooling and was extracted with methylene chloride. The extract was dried over sodium sulfate, followed by distilling off the solvent. The residues was recrystallized from methylenechloride diethyl ether to obtain N-(5-aminovaleryl)-L-phenylalanine 4-benzoylanilide (0.35 g) in the form of a pale yellow solid.

### Example 2

#### Synthesis of N-(6-aminocaproyl)-L-phenylalanine N-methyl-(4-pycoryl)amide dihydrochloride (Compound No. 6)

6-Aminocaproic acid (3 g) and sodium bicarbonate (5 g) were dissolved in water (30 ml) and a toluene solution of benzyl chlorocarbonate (I2.9 g) dissolved in diethyl ether (I0 ml) was dropwise added to the

solution under ice-cooling over 15 minutes, followed by stirring at room temperature for 12 hours. The resultant reaction mixture was washed several times with diethyl ether and was neutralized with 10% hydrochloric acid solution, followed by extracting with ethyl acetate. After the extract was dried over sodium sulfate, the solvent was distilled off. The residue was recrystallized from methylene chloride to obtain 6-benzyloxycarbonylaminocaproic acid (3.9 g) in the form of a white solid.

N-(t-butyloxycarbonyl)-L-phenylalanine (1 g) was dissolved in dry tetrahydrofuran (30 ml) and triethylamine (2 ml) and ethyl chlorocarbonate (0.45 g) were added thereto under ice-cooling, followed by stirring for 30 minutes. To this solution, N-methyl-(4-pycoryl)amine (0.47 g) was added and the mixture was stirred at room temperature for 10 hours. After distilling off the solvent, the resultant reaction mixture was extracted with ethyl acetate and was washed with water, followed by drying over sodium sulfate. The solvent was distilled off and the residue was recrystallized from chloroform. Thus, N-(t-butyloxycarbonyl)-L-phenylalanine N-methyl (4-pycoryl)amide (I) (1.2 g) was obtained in the form of a white solid, which was confirmed by an NMR analysis.

To the compound (I) (0.8 g) obtained above, 4N-hydrogen chloride/dioxane solution (8 ml) was added under ice-cooling, followed by stirring at room temperature for 30 minutes. Diethyl ether (20 ml) was added thereto and the precipitated solid was washed several times with diethyl ether to obtain L-phenylalanine N-methyl-(4-pycoryl)amide dihydrochloride (II) (0.6 g) in the form of a pale yellow solid, which was confirmed by an NMR analysis.

The compound (II) (0.5 g) and 6-benzyloxycarbonylaminocaproic acid (0.39 g) were dissolved in dry pyridine and the condensation agent, 1-ethyl-3-(3-dimethylaminopropyl)carbodiimide hydrochloride (0.4 g) was added under ice-cooling, followed by stirring at room temperature for 12 hours. After removing the solvent, the residue was extracted with methylene chloride, followed by washing with water. After drying over sodium sulfate, the solvent was distilled off and the residue was recrystallized from methylene chloride to obtain N-(6-benzyloxycarbonylaminocaproyl)-L-phenylalanine N-methyl-4-pycorylamide (III) (0.62 g) in the form of a pale yellow solid, which was confirmed by NMR and IR analyses.

The compound (III) (0.6 g) obtained above was added to 30% hydrobromic acid/acetic acid solution (5 ml) under ice-cooling, followed by stirring for 20 minutes. Then, diethyl ether (30 ml) was added and the precipitated solid was washed several times with diethyl ether. A saturated sodium bicarbonate solution (10 ml) was then added under ice-cooling and was extracted with methylene chloride. The extract was dried over sodium sulfate, followed by distilling off the solvent. To the residue, 5% hydrochloride/ethanol solution (5 ml) was added under ice-cooling, followed by stirring for 10 minutes. Then, diethyl ether (30 ml) was added and the resultant precipitated solid was recovered by filtration, followed by washing several times with diethyl ether. Thus, N-(6-aminocaproyl)-L-phenylalanine N-methyl-(4-pycoryl)amide dihydrochloride (0.42 g) in the form of a pale yellow solid was obtained.

## Example 3

### Synthesis of N-[4-(2-aminoethyl)benzoyl]-L-phenylalanine 3-(6-methoxypyridyl)amide dihydrochloride (Compound No. 10)

4-(2-Aminoethyl)benzoic acid (1.46 g) prepared in the manner described in JACS, 65, 2281 (1943) and sodium bicarbonate (1.9 g) were dissolved in water (25 ml) and a toluene solution of benzyl chlorocarbonate (6.02 g) dissolved in diethyl ether (10 ml) was dropwise added to the solution under ice-cooling over 15 minutes, followed by stirring at room temperature for 12 hours. The resultant reaction mixture was washed several times with diethyl ether and was neutralized with 10% hydrochloric acid solution, followed by extracting with ethyl acetate. After the extract was dried over sodium sulfate, the solvent was distilled off. The residue was recrystallized from methylene chloride to obtain 4-(2-benzyloxycarbonylaminoethyl)benzoic acid (2.2 g) in the form of a white solid.

N-(t-butyloxycarbonyl)-L-phenylalanine (1 g) was dissolved in dry tetrahydrofuran (30 ml) and triethylamine (2 ml) and ethyl chlorocarbonate (0.45 g) were added thereto under ice-cooling, followed by stirring for 30 minutes. To this solution, 5-amino-2-methoxypyridine (0.45 g) was added and the mixture was stirred at room temperature for 10 hours. After distilling off the solvent, the resultant reaction mixture was extracted with ethyl acetate and was washed with water, followed by drying over sodium sulfate. The solvent was distilled off and the residue was recrystallized from chloroform. Thus, N-(t-butyloxycarbonyl)-L-phenylalanine 3-(6-methoxypyridyl)amide (I) (1.3 g) was obtained in the form of a pale yellow solid, which

was confirmed by an NMR analysis.

To the compound (I) (0.8 g) obtained above, 4N-hydrogen chloride/dioxane solution (I0 ml) was added under ice-cooling, followed by stirring at room temperature for 30 minutes. Diethyl ether (20 ml) was added thereto and the precipitated solid was washed several times with diethyl ether to obtain L-phenylalanine 3-(6-methoxypyridyl)amide dihydrochloride (II) (0.6 g) in the form of pale yellow solid, which was confirmed by an NMR analysis.

The compound (II) (0.I5), 4-(2-benzyloxycarbonylaminoethyl) benzoic acid (0.I2 g), and triethylamine (I ml) were added to dry pyridine, and after stirring, the condensation agent, I-ethyl-3-(3-dimethylaminopropyl)carbodiimide hydrochloride (0.I6 g) was added under ice-cooling, followed by stirring at room temperature for I2 hours. The reaction mixture was washed with water, acidic water, and alkaline water, followed by drying over sodium sulfate. After drying, the solvent was distilled off to obtain N-{4-(2-benzyloxycarbonylaminoethyl)}benzoyl-L-phenylalanine 3-(6-methoxypyridyl)amide (III) (0.I8 g) in the form of a pale yellow solid, which was confirmed by NMR and IR analyses.

The compound (III) (0.I g) obtained above was added to 30% hydrobromic acid/acetic acid solution (2 ml) under ice-cooling, followed by stirring for 20 minutes. Then, diethyl ether (30 ml) was added and the precipitated solid was washed several times with diethyl ether. A saturated sodium bicarbonate solution (I0 ml) was then added under ice-cooling and was extracted with methylene chloride. The extract was dried over sodium sulfate, followed by distilling off the solvent. To the residue, 5% hydrochloride/ethanol solution (5 ml) was added under ice-cooling, followed by stirring for I0 minutes. Then, diethyl ether (30 ml) was added and the resultant precipitated solid was recovered by filtration, followed by washing several times with diethyl ether. Thus, N-[4-(2-aminoethyl)benzoyl]-L-phenylalanine 3-(6-methoxypyridyl)amide dihydrochloride (0.05 g) in the form of a pale yellow solid was obtained.

## Example 4

### Synthesis of N-(4-amidinobenzoyl)-L-phenylalanine 4-acetylanilide hydroiodic acid (Compound No. II)

N-(t-butyloxycarbonyl)-L-phenylalanine (I g) was dissolved in dry tetrahydrofuran (30 ml) and triethylamine (2 ml) and ethyl chlorocarbonate (0.45 g) were added thereto under ice-cooling, followed by stirring for 30 minutes. To the reaction mixture, 4-acetylaniline (0.5 g) was added, followed by stirring at room temperature for I0 hours. After distilling off the solvent, the reaction mixture was extracted with ethyl acetate and was washed with water, followed by drying over sodium sulfate. After distilling off the solvent, the residue was recrystallized from chloroform to obtain N-(t-butyloxycarbonyl)-L-phenylalanine 4-acetylanilide (I) (I.2 g) in the form of a white solid, which was confirmed by an NMR analysis.

The compound (I) (I.2 g) obtained above was added to 4N-hydrogenchloride/dioxame solution (I0 ml) under ice-cooling, followed by stirring at room temperature for 30 minutes. Diethyl ether (20 ml) was then added and the resultant precipitated solid was washed several times with diethyl ether to obtain L-phenylalanine 4-acetylanilide hydrochloride (II) (I.0 g) in the form of a white solid, which was confirmed by an NMR analysis.

The compound (II) (I.0 g) obtained above, 4-cyanobenzoic acid (0.46 g), and triethylamine (I ml) were added to dry tetrahydrofuran (I0 ml) and the mixture was thoroughly stirred. To the resultant mixture, the condensation agent, I-ethyl-3-(3-dimethylaminopropyl)carbodiimide hydrochloride (0.9 g) was added, followed by stirring at room temperature for I2 hours. The reaction mixture was washed with water, acidic water, and alkaline water, followed by drying over sodium sulfate. After distilling off the solvent, N-(4-cyanobenzoyl)-L-phenylalanine 4-acetylanilide (III) (0.9 g), which was confirmed by an NMR analysis, was obtained in the form of a pale yellow solid.

The compound (III) (0.78 g) obtained above was suspended in a mixed solvent of pyridine (2 ml)-triethylamine (0.5 ml) and the mixture was stirred under ice-cooling for 2 hours, while introducing gaseous hydrogen sulfide. N-(4-thioamidebenzoyl)-L-phenylalanine 4-acetylanilide (IV) (0.68 g), which was confirmed by NMR and IR analyses, was obtained by a conventional post-treatment.

The compound (IV) (0.68 g) obtained above and methyl iodide (2.5 g) were added to acetone (20 ml), followed by stirring at room temperature for 5 hours. After distilling off the solvent, ammonium acetate (0.08

g), ethanol (20 ml) and N,N-dimethylformamide (2 ml) were added to the residue and the mixture was stirred at a temperature of 65 °C for 20 hours. After ice-cooling, after diethylether (50 ml) was added and the precipitated solid was recovered by filtration, followed by washing several times with diethyl ether. Thus, the desired N-(4-amidinobenzoyl)-L-phenylalanine 4-acetylanilide hydroiodide (0.63 g) was obtained.

Example 5

Synthesis of N-(4-amidinobenzoyl)-4-benzyloxy-L-phenylalanine 4-acetylanilide hydroiodic acid (Compound No. 12)

N-(t-butyloxycarbonyl)-4-benzyloxy-L-phenylalanine (1.4 g) was dissolved in dry tetrahydrofuran (30 ml) and triethylamine (2 ml) and ethyl chlorocarbonate (0.45 g) were added thereto under ice-cooling, followed by stirring for 30 minutes. To the reaction mixture, 4-acetylaniline (0.5 g) was added, followed by stirring at room temperature for 10 hours. After distilling off the solvent, the reaction mixture was extracted with ethyl acetate and was washed with water, followed by drying over sodium sulfate. After distilling off the solvent, the residue was recrystallized from chloroform to obtain N-(t-butyloxycarbonyl)-4-benzyloxy-L-phenylalanine 4-acetylanilide (I) (1.6 g) in the form of a white solid, which was confirmed by an NMR analysis.

The compound (I) (1.6 g) obtained above was added to 4N-hydrogenchloride/dioxane solution (10 ml) under ice-cooling, followed by stirring at room temperature for 30 minutes. Diethyl ether (20 ml) was then added and the resultant precipitated solid was washed several times with diethyl ether to obtain 4-benzyloxy-L-phenylalanine 4-acetylanilide hydrochloride (II) (1.3 g) in the form of a white solid, which was confirmed by an NMR analysis.

The compound (II) (1.16 g) obtained above, 4-cyanobenzoic acid (0.49 g), and triethylamine (1 ml) were added to dry tetrahydrofuran (10 ml) and the mixture was thoroughly stirred. To the resultant mixture, the condensation agent, 1-ethyl-3-(3-dimethylaminopropyl)carbodiimide hydrochloride (1.0 g) was added, followed by stirring at room temperature for 12 hours. The reaction mixture was washed with water, acidic water, and alkaline water, followed by drying over sodium sulfate. After distilling off the solvent, N-(4-cyanobenzoyl)-4-benzyloxy-L-phenylalanine 4-acetylanilide (III) (0.9 g), which was confirmed by an NMR analysis, was obtained in the form of a pale yellow solid.

The compound (III) (0.78 g) obtained above was suspended in a mixed solvent of pyridine (2 ml)-triethylamine (0.5 ml) and the mixture was stirred under ice-cooling for 2 hours, while introducing gaseous hydrogen sulfide. N-(4-thioamidebenzoyl)-4-benzyloxy-L-phenylalanine 4-acetylanilide (IV) (0.76 g), which was confirmed by NMR and IR analyses, was obtained by a conventional post-treatment.

The compound (IV) (0.72 g) obtained above and methyl iodide (2.6 g) were added to acetone (20 ml), followed by stirring at room temperature for 5 hours. After distilling off the solvent, ammonium acetate (0.09 g), ethanol (20 ml) and N,N-dimethylformamide (2 ml) were added to the residue and the mixture was stirred at a temperature of 65 °C for 20 hours. After ice-cooling, diethyl ether (50 ml) was added and the precipitated solid was recovered by filtration, followed by washing several times with diethyl ether. Thus, the desired N-(4-amidinobenzoyl)-4-benzyloxy-L-phenylalanine 4-acetylanilide hydroiodide (0.65 g) was obtained.

Example 6

Synthesis of N-(4-guanidinobenzoyl)-L-phenylalanine 4-methylanilide hydrochloride (Compound No. 13)

N-(t-butyloxycarbonyl)-L-phenylalanine (1 g) was dissolved in dry tetrahydrofuran and triethylamine (2 ml) and ethyl chlorocarbonate (0.45 g) were added hereto under ice-cooling, followed by stirring for 30 minutes. To the reaction mixture, 4-methylaniline (0.3 g) was added, followed by stirring at room temperature for further 10 hours. After distilling off the solvent, the reaction mixture was extracted with ethyl acetate and was washed with water, followed by drying over sodium sulfate. After distilling off the solvent, N-(t-butyloxycarbonyl)-L-phenylalanine 4-methylanilide (I) (0.62 g) was obtained in the form of a white solid, which was confirmed by NMR and IR analyses.

The compound (I) (0.6 g) obtained above was added to 4N-hydrogenychloride/dioxane solution (6 ml) under ice-cooling, followed by stirring at room temperature for 30 minutes. Hexane (20 ml) was then added and the resultant precipitated solid was washed several times with diethyl ether to obtain L-phenylalanine 4-methylanilide hydrochloride (II) (0.45 g) in the form of a white solid, which was confirmed by an NMR analysis.

The compound (II) (0.4 g) obtained above and 4-guanidinobenzoic acid hydrochloride (0.2 g), were dissolved in dry pyridine, and under ice-cooling, N,N′-dicyclohexylcarbodiimide (0.2 g) was added and the mixture was allowed to stand at room temperature for l2 hours. Thus, N-(4-guanidinobenzoyl)-L-phenylalanine 4-methylanilide (III) (0.3 g), which was confirmed by an NMR analysis, was obtained in the form of a pale yellow solid by a conventional post-treatment.

The compound (III) (0.3 g) obtained above was added to a saturated sodium bicarbonate solution (l0 ml), and after stirring for 5 minutes, the precipitate was recovered by filtration. The precipitate was washed several times with water to obtain N-(4-guanidinobenzoyl)-L-phenylalanine 4-methylanilide carbonate (IV) (0.l5 g) in the form of a pale yellow solid, which was confirmed by an IR analysis. The compound (IV) (0.l5 g) was added to a 4N-hydrogen chloride/dioxane solution (l ml) under ice-cooling, followed by stirring for 20 minutes. Diethyl ether was then added and the resultant precipitated solid was washed several times with diethyl ether. Thus, the desired N-(4-guanidinobenzoyl)-L-phenylalanine 4-methylanilide hydrochloride (0.l g) was obtained.

## Example 7

### Synthesis of N-(4-guanidinobenzoyl)-L-phenylalanine 4-cis/trans-methylcyclohexylamide hydrochloride (Compound No. 20)

N-(t-butyloxycarbonyl)-L-phenylalanine (l g) was dissolved in dry tetrahydrofuran and triethylamine (2 ml) and ethyl chlorocarbonate (0.4 g) were added thereto under ice-cooling, followed by stirring for 30 minutes. To the reaction mixture, 4-cis/trans-methylcyclohexylamine (0.25 g) was added, followed by stirring at room temperature for a further l0 hours. After distilling off the solvent, the reaction mixture was extracted with ethyl acetate and was washed with water, followed by drying over sodium sulfate. After distilling off the solvent, N-(t-butyloxycarbonyl)-L-phenylalanine 4-cis/trans-methylcyclohexylamide (I) (0.5 g) was obtained in the form of a white solid, which was confirmed by NMR and IR analyses.

The compound (I) (0.5 g) obtained above was added to 4N-hydrogenchloride/dioxane solution (5 ml) under ice-cooling, followed by stirring at room temperature for 30 minutes. Hexane (20 ml) was then added and the resultant precipitated solid was washed several times with diethyl ether to obtain L-phenylalanine 4-cis/transmethylcyclohexylamide hydrochloride (II) (0.45 g) in the form of a white solid, which was confirmed by an NMR analysis.

The compound (II) (0.45 g) obtained above and 4-guanidinobenzoic acid hydrochloride (0.35 g), were dissolved in dry pyridine, and under ice-cooling, N,N-dicyclohexylcarbodiimide (0.36 g) was added and the mixture was allowed to stand at room temperature for l2 hous. Thus, N-(4-guanidinobenzoyl)-L-phenylalanine 4-cis/trans-methylcyclohexylamide (III) (0.5 g), which was confirmed by an NMR analysis, was obtained in the form of a pale yellow solid by a conventional post-treatment.

The compound (III) (0.5 g) obtained above was added to a saturated sodium bicarbonate solution (l0 ml), and after stirring for 5 minutes, the precipitate was recovered by filtration. The precipitate was washed several times with water to obtain N-(4-guanidinobenzoyl)-L-phenylalanine 4-cis/trans-methylcyclohexylamide carbonate (IV) (0.25 g) in the form of a pale yellow solid, which was confirmed by an IR analysis. The compound (IV) (0.2 g) was added to a 4N-hydrogen chloride/dioxane solution (l ml) under ice-cooling, followed by stirring for 20 minutes. Diethyl ether was then added and the resultant precipitated solid was washed several times with diethyl ether. Thus, the desired N-(4-guanidinobenzoyl)-L-phenylalanine 4-cis/trans-methylcyclohexylamide hydrochloride (0.l5 g) was obtained in the form of a pale yellow solid.

## Example 8

Synthesis of N-(4-guanidinobenzoyl)-4-nitro-L-phenylalaninine 4-benzoylanilide methanesulfonate (Compound No. 23)

In an aqueous dioxane solution (200 ml), 4-nitro-L-phenylalanine (8.65 g) and triethylamine (13.5 g) were dissolved and a solution of t-butyloxycarbonyloxyimino-2-phenylacetonitrile (9.49 g) in dioxane (50 ml) was then added thereto, followed by stirring at room temperature for 12 hours. After concentrating in vacuo, water (100 ml) was added to the concentrate and was washed with ethylacetate. The aqueous phase was acidified with 10% citric acid solution, followed by extracting with ethyl acetate. The extract was washed with water, followed by drying over sodium sulfate. The filtrate was concentrated in vacuo and the concentrate was then recrystallized from chloroform-hexane. Thus, N-t-butyloxycarbonyl)-4-nitro-L-phenylalanine (I) (9.05 g) was obtained in the form of a pale yellow solid.

The compound (I) (1.30 g) and triethylamine (3.0 ml) were dissolved in dry tetrahydrofuran (30 ml) and, under ice-cooling, ethyl chlorocarbonate (0.60 g) was added thereto, followed by stirring for 20 minutes. Thereafter, 4-benzoylaniline (0.80 g) was further added, followed by stirring at room temperature for 10 hours. After distilling off the solvent, the residue was extracted with ethyl acetate and was then washed with water, followed by drying over sodium sulfate. The dried extract was concentrated in vacuo to obtain N-(t-butyloxycarbonyl)-4-nitro-L-phenylalanine 4-benzoylanilide (II) (1.00 g) in the form of a white powder, which was confirmed by an NMR analysis.

To the compound (II) (1.00 g) obtained above, 4N-hydrogen chloride/dioxane solution (4 ml) was added, followed by stirring at room temperature for 30 minutes. n-Hexane (30 ml) was added to the resultant solution and the precipitated crystalline product was recovered by filtration. After the product was washed with ethyl ether, the product was dried to obtain 4-nitro-L-phenylalanine 4-benzoylanilide hydrochloride (III) (0.80 g) in the form of a white powder.

The compound (III) (0.50 g). 4-guanidinobenzoic acid hydrochloride (0.26 g), and N,N'-dicyclohexylcarbodiimide (0.28 g) were dissolved in dry pyridine (20 ml) and the mixture was stirred at room temperature for 10 hours. After insoluble matters were filtrated off, the mother liquor was concentrated in vacuo and a saturated aqueous sodium bicarbonate solution was then added, followed by stirring at room temperature for further 10 hours. The insoluble matters were recovered by filtration, washed with water (30 ml), and dried. Thus, N-(4-guanidinobenzoyl)-4-nitro-L-phenylalanine 4-benzoylanilide carbonate (IV) (0.55 g), which was confirmed by an NMR analysis, was obtained in the form of a pale brown powder.

The compound (IV) (0.35 g) obtained above was dissolved in methyl alcohol (10 ml) and methane sulfonic acid (0.07 g) was added thereto at room temperature, followed by stirring for 10 minutes. Furthermore, diethylether (50 ml) was added and the resultant precipitated crystalline product was recovered by filtration. The product was dried to obtain N-(4-guanidinobenzoyl)-4-nitro-L-phenylalanine 4-benzoylanilide methane sulfonate (V) (0.40 g), which was confirmed by an NMR analysis, in the form of a pale yellow powder.

Example 9

Synthesis of N-(4-guanidinobutyryl)-4-nitro-L-phenylalanine 4-benzoylanilide methanesulfonate (Compound No. 30)

The compound (III) (0.63) of Example 8, 4-guanidinobutyric acid (0.22 g), and N,N'-dicyclohexylcarbodiimide (0.35 g) were dissolved in dry pyridine (10 ml) and the mixture was stirred at room temperature for 10 hours. After the insoluble matters were filtered off, diethylether ((50 ml) was added to the mother liquor. The precipitated crystalline product thus formed was recovered by filtration and then suspended in a saturated aqueous sodium carbonate solution, followed by stirring at room temperature for 10 hours. The insoluble product was recovered by filtration, washed with water (30 ml), and dried. The dried product was dissolved in methyl alcohol (5 ml) and methane sulfonic acid (0.17 g) was added thereto at room temperature. The mixture was stirred at room temperature for 10 minutes and diethyl ether (50 ml) was added. The resultant precipitated crystalline product was filtered and dried. Thus, N-(4-guanidinobutyryl)-4-nitro-L-phenylalanine 4-benzoylanilide methane sulfonate (I) (0.60 g), which was confirmed by an NMR analysis, was obtained in the form of a pale yellow powder.

Example I0

Synthesis of N-(trans-4-guanidinomethylcyclohexylcarbonyl)-4-nitro-L-phenylalanine 4-benzoylanilide methane sulfonate (Compound No. 3I)

The compound (III) (0.80 g) off Example 8, trans-4-guanidinomethylcyclohexane carboxylic acid hydrochloride (0.45 g) prepared according to Chem. Pharm. Bull., 33(2), 647 (I985), and N,N'-dicyclohexylcarbodiimide (0.43 g) were dissolved in dry pyridine-N,N'-dimethylformamide solvent (30 ml) and the mixture was stirred at room temperature for I0 hours. After insoluble matters were filtered off, the mother liquor was concentrated in vacuo and a saturated aqueous sodium bicarbonate solution then added, followed by stirring at room temperature for further I0 hours. The insoluble matters were recovered by filtration, washed with water (30 ml) and dried. Thus, N-(trans-4-guanidinomethylcyclohexyl carbonyl)-4-nitro-L-phenylalanine 4-benzoylanilide carbonate (I) (I.I0g), which was confirmed by an NMR analysis, was obtained in the form of a pale yellow powder.

The compound (I) (I.I0 g) obtained above was dissolved in methyl alcohol (8 ml) and methane sulfonic acid (0.I7 g) was added thereto at room temperature, followed by stirring for I0 minutes. Furthermore, diethylether (50 ml) was added and the resultant precipitated crystalline product was recovered by filtration. The precipitated crystalline product was dried to obtain N-(trans-4-guanidinomethylcyclohexylcarbonyl)-4-nitro-L-phenylalanine 4-benzoylanilide methanesulfonate (I.I5 g), which was confirmed by an NMR analysis, in the form of pale yellow powder.

Example II

Synthesis of N-(4-guanidinobenzoyl)-L-phenylalanine 4-hydroxycarbonylmethylanilide hydrochloride (Compound No. 32)

N-(t-butyloxycarbonyl)-L-phenylalanine (3 g) was dissolved in dry tetrahydrofuran (I00 ml) and triethylamine (I3 ml) and ethyl chlorocarbonate (2.7 g) were added thereto under ice-cooling, followed by stirring for 30 minutes. To this reaction mixture, 4-ethoxycarbonylmethylaniline hydrochloride (2.2 g) was added and the mixture was stirred at room temperature for a further I0 hours. After distilling off the solvent, the resultant reaction mixture was extracted with ethyl acetate and was washed with water, followed by drying over sodium sulfate. The solvent was then distilled off. Thus, N-(t-butyloxycarbonyl)-L-phenylalanine 4-ethoxycarbonylmethyl anilide (I) (4.5 g) was obtained in the form of a white powder, which was confirmed by an NMR analysis.

To the compound (I) (I g) obtained above, 4N-hydrogen chloride/dioxane solution (I0 ml) was added under ice-cooling, followed by stirring at room temperature for 30 minutes. N-hexane (30 ml) was added thereto the precipitated solid was washed several times with diethyl ether to obtain L-phenylalanine-4-ethoxycarbonylmethyl anilide hydrochloride (II) (0.5 g) in the form of white solid, which was confirmed by an NMR analysis.

The compound (II) (0.48 g) and 4-guanidino benzoicacid hydrochloride (0.3 g) were dissolved in dry pyridine and N,N'-dicyclohexylcarbodiimide (0.3 g) was added under ice-cooling, followed by allowing to stand at room temperature for I2 hours. After the conventional posttreatment, N-(4-guanidinobenzoyl)-L-phenylalanine 4-ethoxycarbonylmethyl anilide (III) (0.5 g), which was confirmed by an NMR analysis, was obtained.

The compound (III) (0.48 g) obtained above was dissolved in tetrahydrofuran (5 ml) / methanol (8 ml) and an aqueous solution of sodium hydroxide (0.06 g) in water (4 ml) was then added, followed by stirring at a temperature of 40 °C for 2 hours. After distilling off the solvent, the resultant mixture was neutralized with a I0% citric acid solution and the precipitated solid was recovered by filtration. Thus, N-(4-guanidinobenzoyl)-L-phenylalanine 4-hydroxycarbonylmethyl anilide (IV) (0.4 g), which was confirmed by NMR and IR analyses, was obtained in the form of a brown solid.

The compound (IV) (0.4 g) obtained above was added, under ice-cooling, to a 4N-hydrogellchloride/dioxane solution (4 ml), followed by stirring for 20 minutes. To this solution, hexane (30 ml) was added and the product was recovered by filtration, followed by washing several times with diethyl ether. Thus, N-(4-guanidinobenzoyl)-L-phenylalanine 4-hydroxycarbonylmethyl anilide hydrochloride (0.2 g) was obtained in the form of a pale brown solid.

Example I2

Synthesis of N-(4-guanidinobenzoyl)-L-phenylalanine 4-ethoxycarbonylmethyl anilide (Compound No. 34)

N-(t-butyloxycarbonyl)-L-phenylalanine (3 g) was dissolved in dry tetrahydrofuran (I00 ml) and triethyl amine (I3 ml) and ethyl chlorocarbonate (2.7 g) were added thereto under ice-cooling, followed by stirring for 30 minutes. To this solution, 4-ethoxycarbonylmethylaniline hydrochloride (2.2 g) was added and the mixture was stirred at room temperature for I0 hours. After distilling off the solvent, the resultant reaction mixture was extracted with ethyl acetate and was washed with water, followed by drying over sodium sulfate. The solvent was then distilled off. Thus, N-(t-butyloxycarbonyl)-L-phenylalanine 4-ethoxycarbonylmethylanilide (I) (4.5 g) was obtained in the form of a white powder, which was confirmed by an NMR analysis.

To the compound (I) (4.5 g) obtained above, 4N-hydrogen chloride/dioxane solution (I0 ml) was added under ice-cooling, followed by stirring at room temperature for 30 minutes. N-hexane (30 ml) was added thereto and the resultant precipitated solid was washed several times with diethyl ether to obtain L-phenylalanine-4-ethoxycarbonylmethyl anilide hydrochloride (II) in the form of a white solid (0.5 g), which was confirmed by NMR analysis.

The compound (II) (0.48 g) and 4-guanidinobenzoicacid hydrochloride (0.3 g) were dissolved in dry pyridine and N,N'-dicyclohexylcarbodiimide (0.3 g) was added under ice-cooling, followed by allowing to .stand at room temperature for I2 hours. After the conventional posttreatment, N-(4-guanidinobenzoyl)-L-phenylalanine 4-ethoxycarbonylmethylanilide (0.5 g) was obtained.

Example I3

Synthesis of N-(4-guanidinobenzoyl)-4-nitro-L-phenylalanine 4-dimethylcarbamoylbenzylamide methanesulfonate (Compound 8)

4-Aminomethylbenzoic acid (I0 g) and t-butyloxycarbonyloxyimino-2-phenylacetonitrile (I9 g) were dissolved in a water-dioxane mixed solution (200 ml) and triethylamine (I4 g) was then added, followed by stirring at room temperature for I2 hours. After distilling off the solvent, water (I00 ml) was added, followed by washing with ethyl acetate several times. The reaction mixture was acidified with a I0% citric acid solution and then extracted with ethyl acetate, followed by drying over sodium sulfate. After distilling off the solvent, the residue was recrystallized from chloroform. Thus, N-(4-t-butyloxycarbonylaminomethyl)benzoic acid (I) (I4 g), which was confirmed by NMR and IR analyses was obtained in the form of a white solid.

The compound (I) (I.02 g), dimethylamine hydrochloride (0.33 g), and triethylamine (5 ml) were dissolved in dry methylene chloride and the condensation agent, I-ethyl-3-(3-dimethylaminopropyl)-carbodiimide hydrochloride (0.9 g) was then added, followed by stirring at room temperature for I6 hours. The reaction mixture was washed with water, alkaline water, and acidic water, followed by drying over sodium sulfate. After distilling off the solvent, the residue was recrystallized from a mixed solvent of chloroformdiethylether to obtain N-(4-t-butyloxycarbonylaminomethyl)benzoyl dimethylamide (II) (I.I g) in the form of white solid, which was confirmed by NMR and IR analyses. To the compound (II) (0.5 g) obtained above, 4N-hydrogenchloride/dioxane solution (5 ml) was added, followed by stirring at room temperature for 30 minutes. Diethyl ether (30 ml) was added and the precipitated solid was washed with diethyl ether several times. Thus, 4-nitro-L-phenylalanine 4-dimethylcarbamoylbenzylamide hydrochloride (V) (0.I5 g), which was confirmed by an NMR analysis, was obtained in the form of white solid. The compound (V) (0.I5 g) and 4-guanidinobenzoic acid hydrochloride (0.08 g) were dissolved in dry pyridine (20 ml) and the condensation agent, and I-ethyl-3-(3-dimethylaminopropyl)carbodiimide hydrochloride (0.09 g) was then

added, followed by stirring at room temperature for l2 hours. After distilling off the solvent, the residue was washed with a saturated aqueous sodium bicarbonate solution several times to obtain N-(4-guanidinobenzoyl)-4-nitro-L-phenylalanine 4-dimethylcarbamoylbenzylamide carbonate (VI) (0.l6 g) in the form of a pale brown solid. The compound (VI) (0.l6 g) was dissolved in methanol (5 ml) and methanesulfonic acid (0.05 g) was then added, followed by allowing to stand for 30 minutes. Diethyl ether (30 ml) was added and the resultant precipitated solid was washed several times with diethyl ether to obtain N-(4-quauidinobenzoyl)-4-nitro-L-phenylalanine 4-dimethylcarbamoylbenzylamide methanesulfonate (0.l2 g) in the form of a pale brown solid.

Example l4

Synthesis of N-(4-guanidinobenzoyl)-4-benzyloxy-L-phenylalanine 4-(2-methoxycarbonylvinyl)anilide methanesulfonate (Compound No. 33)

4-Aminocinnamic acid (5 g) was added to a saturated hydrogenchloride/methanol solution (50 ml), followed by heating at reflux for 5 hours. After distilling off the solvent, the residue was recrystallized from a mixed solvent of methanol-diethylether to obtain methyl 4-aminocinnamate hydrochloride (I) (6 g) in the form of a pale yellow solid.

The compound (I) (0.4l g), N-(t-butyloxycarbonyl)-4-benzyloxy-L-phenylalanine (0.7 g) and triethylamine (0.3 g) were dissolved in dry N,N-dimethylformamide (20 ml) and the condensation agnet, l-ethyl-3-(3-dimethylamino-propyl)carbodiimide hydrochloride (0.45 g) was then added, followed by stirring at room temperature for l8 horus.

After distilling off the solvent, the resultant reaction mixture was extracted with methylene chloride and was washed with water, alkaline water, and acidic water, followed by drying over sodium sulfate. After distilling off the solvent, the residue was recrystallized from ethanol to obtain N-(t-butyloxycarbonyl)-4-benzyloxy-L-phenylalamine 4-(2-methoxycarbonylvinyl)anilide (II) (0.82 g), which was confirmed by NMR and IR analyses, in the form of a pale brown solid.

To the compound (II) (0.3l g) obtained above, 4N-hydrogen chloride/dioxane solution (5 ml) was added, followed by stirring at room temperature for 30 minutes. Diethylether (30 ml) was added and the precipitated solid was washed with diethylether several times. Thus, 4-benzyloxy-L-phenylalanine 4-(2-methoxycarbonylvinyl)anilide hydrochloride (III) (0.26 g), which was confirmed by NMR and IR analyses, was obtained in the form of a white solid. The compound (III) (0.26 g) and 4-guanidinobenzoic acid hydrochloride (0.l3 g) were dissolved in dry pyridine (20 ml) and the condensation agent, l-ehtyl-3-(3-dimethylaminopropyl)carbodiimide hydrochloride (0.l5 g) was then added, followed by stirring at room temperature for l2 hours. After distilling off the solvent, the residue was washed with a saturated aqueous sodium bicarbonate solution several times to obtain N-(4-guanidinobenzoyl)-4-benzyloxy-L-phenylalanine 4-(2-methoxycarbonylvinyl)anilide carbonate (IV) (0.32 g) in the form of a pale brown solid. The compound (IV) (0.32 g) was dissolved in methanol (5 ml) and methanesulfonic acid (0.05 g) was then added, followed by allowing to stand for 30 minutes. Diethylether (30 ml) was added and the precipitated solid was washed several times with diethylether to obtain N-(4-guanidinobenzoyl)-4-benzyloxy-L-phenylalanine 4-(2-methoxycarbonylvinyl)anilide methanesulfonate (0.26 g) in the form of a pale brown solid.

The phenylalanine derivatives or the salts thereof according to the present invention, which are an effective component of the proteinase inhibitor of the present invention, have very potent inhibition activities against proteinases such as plasmin, kallikrein, trypsin, and urokinase, as shown in the below-mentioned test results. The plasmin inhibition activity is different from the effect exhibited by the antiplasmins of the prior art, when contrasted with known drugs of the prior art such as tranexamic acid or ε-aminocaploic acid which selectively inhibits only plasmin among proteinases. For example, some effective ingredients of the proteinase inhibitor according to the present invention exhibit an inhibition activity against urokinase, which is a plasminogen activating enzyme, as is well known. Accordingly, the inhibition of this enzyme can provide preferable hemostatics. On the other hand, some of the proteinase inhibitors according to the present invention exhibit antikallikrein activity and antitrypsin activity. Accordingly that these inhibition activities can provide, together with the antiplasmin activity, a strong anti-inflammatory agent.

Furthermore, the antitrypsin activity is effective in the curing or treatment of pancreatitis. The compounds according to the present invention have low molecular weight, compared with aprotinin available as a proteinase inhibitor, and therefore, are easy to supply with high quality. Furthermore, the present

compounds have remarkably high activities, even when compared with the known phenylalanine derivatives having the structure similar to that of the present invention as are illustrated in the Table 3.

In the following, the antiplasmin activity, antikallikrein activity, antitrypsin activity, anti-urokinase activity and antithrombin activity of the present compounds are described in detail by referring to typical test examples.

The measurement methods employed in the following test examples are as described below. The test results are shown in Table 2 by referring to the compound Nos. in the above Table I for the compounds of the present invention, and the test results are shown in Table 4 by showing the structures of the compounds in Table 3 for the commercially available antiplasmins as Comparative Examples.

(I) Evaluation of Antiplasmin Activity

(i) Determination of inhibition activity on fibrin decomposition

An inhibitor sample is dissolved in a 0.l8 M borate-physiological salt buffer solution (pH = 7.4) to bring the total volume to 600 µl. To this buffer solution, 200 µl of a 0.2% bovine fibrinogen, l00 µl of a 0.3 casein unit/ml human plasmin solution, and l00 µl of a 50 unit/ml bovine thrombin solution, all dissolved in the above-mentioned buffer, are added at a temperature of 37 °C in a constant temperature bath. The dissolution time of the fibrin mass formed above is then determined. Thus, the concentration $I_{50}$ of the inhibitor sample (i.e., concentration for 50% inhibition, µmol), at which the dissolution time obtained in the absence of the inhibitor (i.e., about 5 minutes) is extended twice, is determined.

(ii) Determination of inhibition activity on S-225l decomposition

An inhibitor sample is dissolved in a 0.05 M Tris-hydrochloric acid buffer solution (pH = 7.4) to bring the total volume to 400 µl. To this solution, 50 µl of a 3 mM S-225l solution is added and the mixture is incubated at a temperature of 37 °C for 5 minutes in a constant temperature bath. Then 50 µl of a 0.2 casein unit/ml human plasmin is added and the mixture is incubated at a temperature of 37 °C for 4 minutes. Thereafter, the reaction is stopped by adding 50 µl of 50% acetic acid.

The absorbance of p-nitroaniline formed in the reaction mixture is determined at 405 nm. Thus, the concentration $I_{50}$ (µmol) of the inhibitor sample, at which the absorbance is one half (i.e., l/2) of that obtained in the absence of the inhibitor, is determined.

(iii) Determination of inhibition activity on fibrinogendecomposition

An inhibitor sample is dissolved in a 0.l8 M borate-physiological salt buffer solution (pH = 7.4) to bring the total volume to 400 µl. To this solution, 500 µl of a 0.4% bovine fibrinogen solution and l00 µl of a l casein unit/ml of human plasmin solution, all dissolved in the above-mentioned buffer, are added at a temperature of 37 °C in a constant temperature bath. After the mixture is allowed to stand at a temperature of 37 °C for l0 minutes, 3800 µl of the above-mentioned buffer containing l3.2 mM of tranexamic acid and 200 µl of a 50 unit/ml of bovine thrombin solution are added to terminate the reaction. The mixture is then incubated at a temperature of 37 °C for l5 minutes to form the fibrin. The fibrin clot thus formed is adhered to or wound around a glass rod and is washed with water. The amount of fibrinogen remaining is determined according to a tyrosine coloring method using a phenol reagent (see J. Biol. Chem., 73, 627 (l927)). From the thus-determined, amount of fibrinogen remaining the amount of decomposed fibrinogen is calculated. Thus, the concentration $I_{50}$ (µmol) of the inhibitor sample, at which the amount of decomposed fibrinogen is one half (i.e., l/2) of that obtained in the absence of the inhibitor sample, is determined.

(2) Evaluation of Antithrombin Activity

(i) Determination of inhibition activity against fibrin formation

An inhibitor sample is dissolved in a 0.I8 M borate-physiological salt buffer solution (pH = 7.4) to bring the total volume to 500 µl. To this solution, 400 µl of a 0.2% bovine fibrinogen solution and I00 µl of a 4 unit/ml bovine thrombin solution are added at a temperature of 37°C in a constant temperature bath. Thus, a coagulation time is determined. The inhibitor concentration $I_{50}$ (µmol), at which the coagulation time obtained in the absence of the inhibitor is extended twice, is determined.

(ii) Determination of inhibition activity on S-2238 decomposition

An inhibitor sample is dissolved in a 0.05 M Tris-hydrochloric acid buffer solution (pH = 8.3) to bring the a total volume to 400 µl. To this solution, 50 µl of a 0.2 mM S-2238 solution is added and the mixture is incubated at a temperature of 37°C for 5 minutes in a constant temperature bath. Then 50 µl of a 0.2 unit/ml bovine thrombin solution is added thereto and the absorbance, at 405 nm, of the p-nitroaniline formed per minute is determined at a temperature of 37°C by using the so-called initial velocity method. Thus, the concentration $I_{50}$ (µmol) of the inhibitor sample, at which the absorbance is one half (i.e., I/2) of that obtained in the absence of the inhibitor sample, is determined.

(3) Evaluation of Antitrypsin Activity

Determination of inhibition activity against S-2238 decomposition

An inhibitor sample is dissolved in a 0.05 M Tris-imidazole buffer solution (pH = 8.I) and I25 µl of a I mM S-2238 solution is added to bring the total volume to I.20 ml. The mixture is incubated at a temperature of 37°C for 5 minutes in a constant temperature bath. To this mixture, 0.05 ml of bovine trypsin is added and the absorbance, at 405 nm, of the p-nitroaniline formed per minute is determined at a temperature of 37°C by using the so-called initial velocity method. Thus, the concentration $I_{50}$ (µmol) of the inhibitor sample, at which the absorbance is one half (i.e., I/2) of that obtained in the absence of the inhibitor sample, is determined.

(4) Evaluation of Anti-Plasma Kallikrein Activity

Determination of inhibition activity on S-2302 decomposition

An inhibitor sample is dissolved in a 0.05 M Tris-hydrochloric acid buffer solution (pH = 7.8) to bring the total volume to 400 µl. To this solution, 50 µl of a 2mM S-2302 solution is added and the mixture is incubated at a temperature of 37°C for 5 minutes in a constant temperature bath. Then, 50 µl of a 0.I2 unit/ml human plasma kallikrein is added and the mixture is incubated at a temperature of 37°C for 5 minutes. Thereafter, 50 µl of 50% acetic acid is added to terminate the reaction. The absorbance of the p-nitroaniline formed in the reaction mixture is measured at 405 nm. Thus, the concentration $I_{50}$ (µmol) of the inhibitor sample, at which the absorbance is one half (i.e., I/2) of that obtained in the absence of the inhibitor sample, is determined.

(5) Evaluation of Antiurokinase Activity

Determination of inhibition activity for S-2444 decomposition

An inhibitor sample is dissolved in a 0.05 M Tris-hydrochloric acid buffer solution (pH = 8.8) to bring the total volume to 400 µl. To this solution, 50 µl of a l mM S-2444 solution is added and the mixture is incubated at a temperature of 37° C for 5 minutes in a constant temperature bath. Then 50 µl of a 500 unit/ml human urokinase is added and the mixture is incubated at a temperature of 37° C for 5 minutes. Thereafter, 50 µl of 50% acetic acid is added to terminate the reaction. The absorbance of the p-nitroaniline formed in the reaction mixture is measured at 405 nm. Thus, the concentration $I_{50}$ (µmol) of the inhibitor sample, at which the absorbance is one half (i.e., I/2) of that obtained in the absence of the inhibitor sample, is determined.

When the compounds of the present invention are used as a medicine, there are no critical limitations to the administration methods. The present proteinase inhibitor can be formulated by any conventional method in pharmaceutics. For example, the present proteinase inhibitor may be applied in any conventional manner including intravenous injection, intramuscular injection, instillation, oral administration, respiratory inhalation, instillation, rhinenchysis, and external skin treatment. Although there are no critical limitations to the administration dosage, the suitable dosage is l to l000 mg/day/person, which can be conveniently decreased or increased as desired, as a matter of course.

Table 2

| Compound No. | Plasmin | | Thrombin | | Trypsin | Plasma Kallikrein | Urokinase |
|---|---|---|---|---|---|---|---|
| | S-2251 | Fibrin | S-2238 | Fibrinogen | S-2238 | S-2302 | S-2444 |
| 1 | 23 | | >200 | | | | |
| 2 | 120 | >100 | >200 | >100 | 75 | | |
| 3 | >1000 | >1000 | >1000 | >1000 | | | >1000 |
| 4 | 39 | | | | >200 | | |
| 5 | >500 | >500 | >500 | >500 | | >500 | >500 |
| 6 | >1000 | >1000 | >500 | | | >1000 | >500 |
| 7 | >100 | | >180 | >120 | | >120 | 120 |
| 9 | 30 | | | | 16 | 31 | 100 |
| 10 | 830 | 970 | | | | 85 | |
| 11 | 23 | 21 | | | 3.6 | 0.8 | 13 |
| 12 | 2.2 | 0.98 | | | 0.75 | 0.3 | 4.0 |
| 13 | >1000 | >200 | | | 1.3 | 4.2 | 420 |
| 14 | | | >500 | >100 | 13 | 5.9 | 640 |
| 15 | >400 | >200 | 330 | >500 | 14 | 2.2 | 190 |
| 16 | | | | | 1.5 | 3.6 | 40 |
| 17 | | | >100 | >200 | 370 | | |
| 18 | | | >1000 | >1000 | 35 | 12 | 630 |
| 19 | >1000 | >1000 | | >1000 | | 1000 | |
| 20 | 140 | 73 | | >100 | 15 | 45 | 310 |
| 21 | 450 | | 400 | >100 | 6.0 | 15 | 740 |
| 22 | >1000 | >1000 | >1000 | >1000 | | >1000 | >1000 |
| 23 | 89 | | | | 0.43 | 11 | 11 |
| 24 | 21 | 16 | | | 9.1 | 5.7 | 240 |
| 25 | 160 | 48 | | >100 | 17 | 12 | |
| 26 | 63 | | >100 | | 70 | 25 | 160 |
| 27 | 31 | | | | | 90 | |
| 28 | 140 | | | | 110 | 170 | |
| 29 | 120 | 82 | | | 17 | 56 | |
| 30 | 60 | | | | | 23 | |
| 31 | >100 | | | | | >100 | >100 |

## Table 3

| No. | Compound |
|---|---|
| 1 | 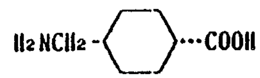 ( t - AMCHA ) |
| 2 | $H_2N(CH_2)_5COOH$ ( EACA ) |
| 3 | 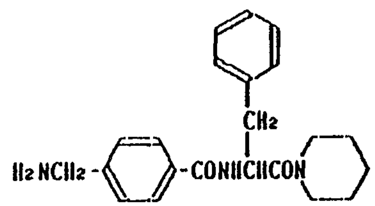 · HCl |
| 4 | 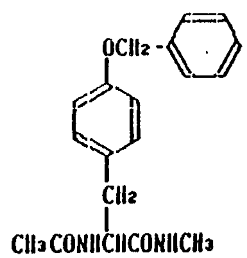 |

| No. | Compound |
|---|---|
| 5 | 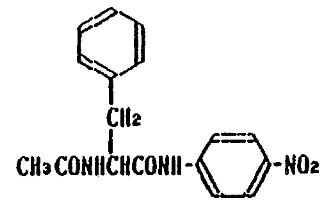 |
| 6 | 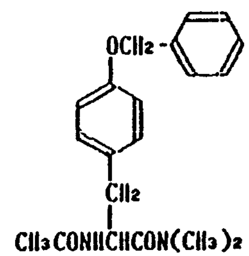 |
| 7 | 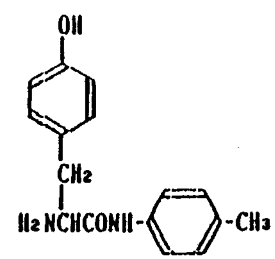 |

<u>Table 4</u>

| Compound No. | Plasmin | | Thrombin | | Trypsin | Plasma Kallikrein | Urokinase |
|---|---|---|---|---|---|---|---|
| | S-2251 | Fibrin | S-2238 | Fibrinogen | S-2238 | S-2302 | S-2444 |
| 1 | 75,000 | 60 | >1,000 | >1,000 | >1,000 | >1,000 | >1,000 |
| 2 | 180,000 | 200 | | | | | |
| 3 | >1,000 | >1,000 | >1,000 | >1,000 | >300 | >1,000 | >1,000 |
| 4 | >200 | >200 | >200 | >200 | | >200 | >200 |
| 5 | >100 | >100 | >100 | >100 | >150 | >100 | >100 |
| 6 | >200 | >200 | >200 | >200 | | >200 | >200 |
| 7 | >1,000 | >1,000 | >1,000 | >1,000 | >300 | >1,000 | >1,000 |

## Claims

1. A phenylalanine derivative having the formula (I):

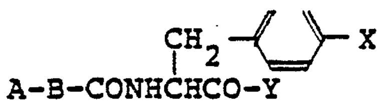 (I)

wherein A represents

(a) $H_2N-$, (b) 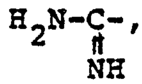, or (c) 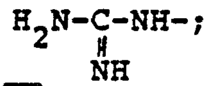;

B represents (a) 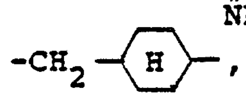,

(b) 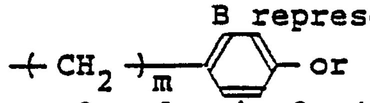 or (c) $-(CH_2)_n-$, wherein m is 0, 1, 2 or 3 and n is 3, 4, or 5, provided that A-B is not 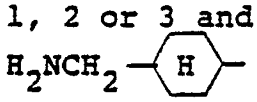, 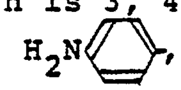, and 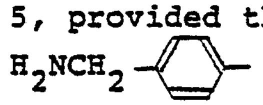;

X represents (a) hydrogen, (b) hydroxy, (c) nitro, or (d) $C_1$-$C_4$ alkyloxy which may be substituted with phenyl;

Y represents

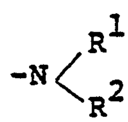

wherein $R^1$ and $R^2$ are, independently, (a) hydrogen provided that both $R^1$ and $R^2$ are not hydrogen at the same time; (b) phenyl which may be substituted with (i) phenylcarbonyl, (ii) $C_1$-$C_4$ alkylcarbonyl, (iii) $C_1$-$C_4$ alkyl which may further be substituted with $C_1$-$C_4$ -alkoxycarbonyl or hydroxycarbonyl, (iv) $C_2$-$C_5$ alkenyl which may be further substituted with $C_1$-$C_4$ alkoxycarbonyl, (v) trifluoromethyl; (c) pyridyl which may be substituted with $C_1$-$C_4$ alkoxy; (d) $C_1$-$C_4$ alkyl which may be substituted with (i) hydroxy, (ii) $C_1$-$C_4$ alkoxycarbonyl, (iii) pyridyl, or (iv) phenyl which may further be substituted with $C_1$-$C_4$ alkylaminocarbonyl; (e) $C_5$-$C_7$ cycloalkyl which may be substituted with $C_1$-$C_4$ alkyl; or (f) $R^1$ and $R^2$ may form, with the nitrogen atom attached thereto, (i) piperidino which may be substituted with phenylcarbonyl or (ii) pyrrolidyl which may be substituted with $C_1$-$C_4$ alkoxycarbonyl; or a pharmaceutically acceptable salt thereof.

2. A phenylalanine derivative as claimed in claim 1, wherein the pharmaceutically acceptable salt is at least one salt selected from the group consisting of hydrochloride, hydrobromide, hydroiodide, sulfate, nitrate, phosphate, oxalate, succinate, glycolate, malate, citrate, lactate, benzene sulfonate, toluene sulfonate, and methane sulfonate.

3. A proteinase inhibitor comprising as an essential component the phenylalanine derivative of claim 1 or the pharmaceutically acceptable salt thereof.

4. A proteinase inhibitor as claimed in claim 3, wherein the pharmaceutically acceptable salt is at least one salt selected from the group consisting of hydrochloride, hydrobromide, hydroiodide, sulfate, nitrate, phosphate, oxalate, succinate, glycolate, malate, citrate, lactate, benzene sulfonate, toluene sulfonate, and methane sulfonate.

5. A phenylalanine derivative as claimed in claim 1, wherein A and B in the formula (I) represent as its combination,

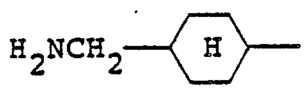 , 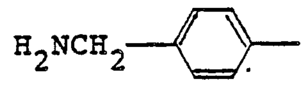 , $H_2N(CH_2)_{3-5}-$,

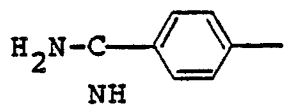 , 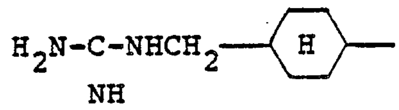 ,

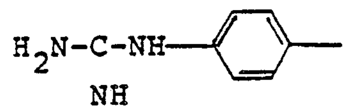, or 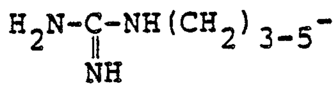

6. A phenylalanine derivative as claimed in claim I, wherein in the formula (I) A and B represent as its combination,

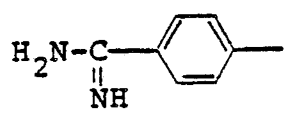 , or 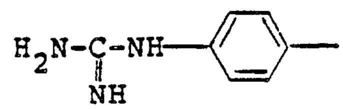 ;

X represent hydrogen, nitro or benzyloxy; and Y represent -NHR wherein R is (a) phenyl which may be substituted with (i) phenylcarbonyl, (ii) $C_1$-$C_4$ alkylcarboxyl, (iii) $C_1$-$C_3$ alkyl which may further be substituted with $C_1$-$C_2$ alkoxycarbonyl or hydroxycarbonyl, (iv) $C_2$ alkenyl which may further be substituted with $C_1$-$C_2$ alkoxycarbonyl; (b) pyridyl which may be substituted with $C_1$ alkoxy; or (c) $C_1$-$C_4$ alkyl which may be substituted with pyridyl, or phenyl which may further be substituted with di $C_1$ alkylaminocarbonyl; or piperidino which may be substituted with phenylcarbonyl or a pharmaceutically acceptable salt thereof.

7. A proteinase inhibitor comprising as an essential compounent the phenylalanine derivative of claim 6 or the pharmaceutically acceptable salt thereof.

8. N-(4-guanidinobenzoyl)-4-nitro-L-phenylalanine 4-dimethylcarbamoylbenzylamide or its salts.

9. N-(4-amidinobenzoyl)-L-phenylalanine 4-acetylanilide or its salts.

I0. N-(4-amidinobenzoyl)-4-benzyloxy-L-phenylalanine 4-acetylanilide or its salts.

II. N-(4-guanidinobenzoyl)-L-phenylalanine 4-acetylanilide or its salts.

I2. N-(4-guanidinobenzoyl)-L-phenylalanine 3-(6-methoxypyridyl)amide or its salts.

I3. N-(4-guanidinobenzoyl)-4-nitro-L-phenylalanine 4-benzoylanilide or its salts.

I4. N(4-guanidinobenzoyl)-4-benzyloxy-L-phenylalanine 4-benzoyl pyperidinoamide or its salts.

I5. N-(4-guanidinobenzoyl)-L-phenylalanine 4-hydroxycarbonylmethylanilide or its salts.

I6. N-(4-guanidinobenzoyl)-4-benzyloxy-L-phenylalanine 4-(2-methoxycarbonylvinyl)anilide or its salts.

European Patent Office

# EUROPEAN SEARCH REPORT

Application Number

EP 87 10 4691

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | CHEMICAL ABSTRACTS, vol. 101, no. 1, 2nd July 1984, page 657, abstract no. 7612p, Columbus, Ohio, US; B. VOIGT "Preparation of different N-alpha-arylsulfonylated or benzoylated amino acid amides with aromatic aminomethyl groups" & Pharmazie 1984, 39(1), 68-69 --- | 1 | C 07 C 129/12<br>C 07 C 103/80<br>C 07 C 103/84<br>C 07 C 123/00<br>C 07 D 213/32<br>C 07 D 213/40<br>C 07 D 213/75<br>C 07 D 295/18 |
| A | DE-B-2 528 730 (PFIZER)<br>* claims * ----- | 1 | |
| | | | TECHNICAL FIELDS SEARCHED (Int. Cl.4) |
| | | | C 07 C 123/00<br>C 07 C 129/00<br>C 07 C 103/00 |

The present search report has been drawn up for all claims

| Place of search | Date of completion of the search | Examiner |
|---|---|---|
| BERLIN | 15-11-1987 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)